# EUROPEAN PATENT APPLICATION

(11) **EP 4 806 917 A1**
(43) Date of publication of application: **16.09.2026**
(21) Application number: 23957931.1
(22) Date of filing: 08.11.2023
(51) Int. Cl.: C07K 16/28, C07K 19/00, C12N 15/13, C12N 5/10, C12N 15/867, A61K 39/395, A61K 39/00, A61P 35/00

(54) **ANTIGEN BINDING PROTEIN TARGETING B7H3 AND USE THEREOF**

(71) Applicant: Chongqing T-Maximum Life and Health Technology Co., Ltd, Chongqing 401320 (CN)
(72) Inventor: SHANG, Xiaoyun, Chongqing 401320 (CN); WANG, Xiaoping, Chongqing 401320 (CN); YANG, Yandong, Chongqing 401320 (CN); GAO, Wei, Chongqing 401320 (CN); WANG, Zhihong, Chongqing 401320 (CN); TANG, Yao, Chongqing 401320 (CN)
(74) Representative: Elzaburu S.L.P.
(86) International application number: PCT/CN2023/130359
(87) International publication number: WO 2025/097322

(57) **Abstract**

An isolated antigen binding protein, which can specifically bind to B7H3. Further provided are a method for preparing the isolated antigen binding protein and the use thereof.

## Description

### TECHNICAL FIELD

The present application relates to the field of biomedicine, and in particular, to an antigen-binding protein targeting B7H3.

### BACKGROUND

B7H3, also referred to as CD276, belongs to the B7 family of immunoregulatory proteins and is a type I membrane protein with an extracellular domain sequence similar to those of other B7 family members. The B7H3 gene is located on human chromosome 15 and consists of ten exons, of which exons 4 to 7 encode extracellular IgV-IgC domains. Although B7H3 mRNA is widely expressed in normal tissues, the expression levels of B7H3 protein are extremely low or absent in normal tissues, suggesting that the expression of B7H3 protein undergoes strict post-transcriptional regulation. In contrast, the B7H3 protein is overexpressed in various malignant tumors and is associated with poor prognosis, higher tumor grade and tumor metastasis, drug resistance, and low overall survival.

In recent years, more and more studies have found that aberrant expression of B7H3 is present in various tumor tissues, such as ovarian cancer, breast cancer, lung cancer, kidney cancer, colorectal cancer, and prostate cancer. Clinical retrospective case analyses have revealed that the expression of B7H3 is correlated with clinicopathological characteristics. Survival analyses have shown that cases with high expression of B7H3 have poor prognosis and high mortality. In addition, high levels of soluble B7H3 (sB7H3) have also been detected in the peripheral blood of tumor patients. Furthermore, studies have also found that B7H3 is associated with transplant immune rejection and autoimmune pathology. Grafts transfected with the B7H3 gene can inhibit the activity of host T cells, thereby prolonging the survival time of the grafts. These research results indicate that B7H3 is an important co-inhibitory molecule and has significant research and application value.

There is an urgent need in the art to develop new and effective specific antibodies targeting B7H3.

### SUMMARY

The present application provides an isolated antigen-binding protein targeting B7H3, which has one or more of the following properties: 1) capable of specifically binding to B7H3; 2) having strong affinity for B7H3; 3) capable of being used to detect the expression level of B7H3; 4) capable of being used to diagnose a B7H3-related disease; and 5) capable of being used to prevent and/or treat a disease and/or condition. The present application further provides a polypeptide molecule comprising said isolated antigen-binding protein; a chimeric antigen receptor comprising said isolated antigen-binding protein; and an immunoconjugate comprising said isolated antigen-binding protein; a nucleic acid molecule encoding said isolated antigen-binding protein; an expression vector; a host cell; an immunoconjugate and a pharmaceutical composition comprising the antigen-binding protein; a method for preparing said isolated antigen-binding protein; and use of said isolated antigen-binding protein described in the present application.

In one aspect, the present application provides an isolated antigen-binding protein capable of specifically binding to B7H3, wherein the isolated antigen-binding protein comprises HCDR1, HCDR2, and HCDR3 as defined in any one of the following groups (a) to (d):
a) the HCDR1 comprising the amino acid sequence set forth in SEQ ID NO: 1, the HCDR2 comprising the amino acid sequence set forth in SEQ ID NO: 2, and the HCDR3 comprising the amino acid sequence set forth in SEQ ID NO: 3;
b) the HCDR1 comprising the amino acid sequence set forth in SEQ ID NO: 7, the HCDR2 comprising the amino acid sequence set forth in SEQ ID NO: 8, and the HCDR3 comprising the amino acid sequence set forth in SEQ ID NO: 9;
c) the HCDR1 comprising the amino acid sequence set forth in SEQ ID NO: 12, the HCDR2 comprising the amino acid sequence set forth in SEQ ID NO: 13, and the HCDR3 comprising the amino acid sequence set forth in SEQ ID NO: 3; or
d) the HCDR1 comprising the amino acid sequence set forth in SEQ ID NO: 14, the HCDR2 comprising the amino acid sequence set forth in SEQ ID NO: 15, and the HCDR3 comprising the amino acid sequence set forth in SEQ ID NO: 16.

In certain embodiments, the isolated antigen-binding protein comprises LCDR1, LCDR2, and LCDR3 as defined in any one of the following groups (a) to (c):
a) the LCDR1 comprising the amino acid sequence set forth in SEQ ID NO: 4, the LCDR2 comprising the amino acid sequence set forth in SEQ ID NO: 5, and the LCDR3 comprising the amino acid sequence set forth in SEQ ID NO: 6;
b) the LCDR1 comprising the amino acid sequence set forth in SEQ ID NO: 10, the LCDR2 comprising the amino acid sequence set forth in SEQ ID NO: 11, and the LCDR3 comprising the amino acid sequence set forth in SEQ ID NO: 6; or
c) the LCDR1 comprising the amino acid sequence set forth in SEQ ID NO: 17, the LCDR2 comprising the amino acid sequence set forth in SEQ ID NO: 18, and the LCDR3 comprising the amino acid sequence set forth in SEQ ID NO: 19.

In certain embodiments, the isolated antigen-binding protein comprises a heavy chain variable region (VH), and the VH comprises the amino acid sequence set forth in SEQ ID NO: 20.

In certain embodiments, the isolated antigen-binding protein comprises a light chain variable region (VL), and the VL comprises the amino acid sequence set forth in SEQ ID NO: 21.

In certain embodiments, the isolated antigen-binding protein comprises a heavy chain variable region having the amino acid sequence set forth in SEQ ID NO: 20 and a light chain variable region having the amino acid sequence set forth in SEQ ID NO: 21.

In certain embodiments, the isolated antigen-binding protein comprises a heavy chain constant region, and the heavy chain constant region thereof may be derived from a human or rabbit antibody heavy chain constant region.

In certain embodiments, the isolated antigen-binding protein comprises a heavy chain constant region, and the heavy chain constant region may be derived from an IgG heavy chain constant region.

In certain embodiments, the heavy chain constant region of the isolated antigen-binding protein may comprise the amino acid sequence set forth in SEQ ID NO: 22.

In certain embodiments, the isolated antigen-binding protein comprises a light chain constant region, and the light chain constant region thereof may be derived from a human or rabbit.

In certain embodiments, the isolated antigen-binding protein comprises a light chain constant region, and the light chain constant region thereof may be derived from a human or rabbit κ chain or λ chain.

In certain embodiments, the light chain constant region thereof may comprise the amino acid sequence set forth in SEQ ID NO: 23.

In certain embodiments, the isolated antigen-binding protein comprises an antibody heavy chain HC, and the HC comprises a heavy chain having the amino acid sequence set forth in SEQ ID NO: 24.

In certain embodiments, the isolated antigen-binding protein comprises an antibody light chain LC, and the LC comprises a light chain having the amino acid sequence set forth in SEQ ID NO: 25.

In certain embodiments, the isolated antigen-binding protein comprises an antibody or an antigen-binding fragment thereof.

In certain embodiments, the antigen-binding fragment comprises a Fab, a Fab', an Fv fragment, an F(ab')2, an F(ab)2, an scFv, a di-scFv, and/or a dAb.

In certain embodiments, when the antigen-binding protein is an scFv, the heavy and light chain variable regions thereof may comprise a linker peptide therebetween.

In certain embodiments, the antibody is one or more selected from the group consisting of: a monoclonal antibody, a rabbit antibody, a chimeric antibody, a humanized antibody, a recombinant antibody, a single-chain antibody, a bifunctional antibody, a trifunctional antibody, and a tetrafunctional antibody.

In certain embodiments, the chimeric antibody comprises a human, goat, monkey, dog, mouse, or rabbit chimeric antibody.

In certain embodiments, the antigen-binding protein is an IgG antibody.

In certain embodiments, the antigen-binding protein is labeled.

In certain embodiments, the label comprises a fluorescent label, an enzymatic label, a biotin label, or a radioactive label.

In another aspect, the present application provides a polypeptide molecule comprising the isolated antigen-binding protein described herein.

In another aspect, the present application provides a chimeric antigen receptor comprising the isolated antigen-binding protein described herein.

In another aspect, the present application provides an immunoconjugate comprising the isolated antigen-binding protein described herein.

In another aspect, the present application provides a nucleic acid molecule encoding the isolated antigen-binding protein described herein, the polypeptide molecule described herein, or the chimeric antigen receptor described herein.

In another aspect, the present application provides a vector comprising the nucleic acid molecule described herein.

In another aspect, the present application provides a cell comprising the isolated antigen-binding protein described herein, the chimeric antigen receptor described herein, the nucleic acid molecule described herein, or the vector described herein.

In another aspect, the present application provides a pharmaceutical composition comprising the isolated antigen-binding protein described herein, the polypeptide molecule described herein, the chimeric antigen receptor described herein, the immunoconjugate described herein, the nucleic acid molecule described herein, the vector described herein, and/or the cell described herein, and a pharmaceutically acceptable carrier.

In another aspect, the present application provides a reagent comprising the isolated antigen-binding protein described herein, the polypeptide molecule described herein, the chimeric antigen receptor described herein, the immunoconjugate described herein, the nucleic acid molecule described herein, the vector described herein, the cell described herein, and/or the pharmaceutical composition described herein.

In another aspect, the present application provides an assay plate comprising the isolated antigen-binding protein described herein, the polypeptide molecule described herein, the chimeric antigen receptor described herein, the immunoconjugate described herein, the nucleic acid molecule described herein, the vector described herein, the cell described herein, and/or the pharmaceutical composition described herein.

In another aspect, the present application provides a kit comprising the isolated antigen-binding protein described herein, the polypeptide molecule described herein, the chimeric antigen receptor described herein, the immunoconjugate described herein, the nucleic acid molecule described herein, the vector described herein, the cell described herein, the pharmaceutical composition described herein, the reagent described herein, and/or the assay plate described herein.

In certain embodiments, the kit comprises a negative control reagent and/or material, and/or a positive control reagent and/or material.

In another aspect, the present application provides a method for preparing the isolated antigen-binding protein described herein.

In certain embodiments, the method comprises the following steps:
a) culturing the cell described herein; and/or
b) isolating an antigen-binding protein from a culture, wherein the antigen-binding protein is the antigen-binding protein described herein.

In another aspect, the present application provides a method for detecting B7H3 in a sample, which comprises using the isolated antigen-binding protein described herein, the polypeptide molecule described herein, the chimeric antigen receptor described herein, the immunoconjugate described herein, the nucleic acid molecule described herein, the vector described herein, the cell described herein, the pharmaceutical composition described herein, the reagent described herein, the assay plate described herein, and/or the kit described herein. In another aspect, the present application provides use of the isolated antigen-binding protein described herein, the polypeptide molecule described herein, the chimeric antigen receptor described herein, the immunoconjugate described herein, the nucleic acid molecule described herein, the vector described herein, the cell described herein, and/or the pharmaceutical composition described herein in preparing a medicament for use in preventing and/or treating a disease and/or condition.

In certain embodiments, the disease and/or condition includes a B7H3-related disease and/or condition.

In certain embodiments, the disease and/or condition includes a tumor.

In certain embodiments, the disease and/or condition includes adrenocortical carcinoma, bladder cancer, breast cancer, cholangiocarcinoma, colorectal cancer, lymphoma, esophageal cancer, brain glioma, neuroblastoma, head and neck squamous cell carcinoma, kidney cancer, liver cancer, lung cancer, ovarian cancer, pancreatic cancer, prostate cancer, sarcoma, melanoma, gastric cancer, thymus cancer, endometrial cancer, and/or cervical cancer.

In another aspect, the present application provides use of the isolated antigen-binding protein described herein, the polypeptide molecule described herein, the chimeric antigen receptor described herein, the immunoconjugate described herein, the nucleic acid molecule described herein, the vector described herein, the cell described herein, and/or the pharmaceutical composition described herein in preparing a detection reagent.

In certain embodiments, the detection reagent is used for:
a) detecting B7H3 in a sample;
b) detecting endogenous B7H3 protein in tumor cells;
c) detecting tumor cells expressing B7H3;
d) detecting expression level of B7H3 in a biological sample from a patient to predict or determine prognosis and/or progression of a disease and/or condition;
e) detecting expression level of B7H3 in a biological sample from a patient to determine a patient population for administration of a B7H3-targeted therapy, wherein when the expression level of B7H3 is medium or high, the B7H3-targeted therapy is administered to the patient;
f) detecting expression level of B7H3 in a biological sample from a patient to predict or determine efficacy of a B7H3-targeted therapy administered to the patient, wherein when the expression level of B7H3 is medium or high, it indicates that the B7H3-targeted therapy is therapeutically effective; and/or
g) detecting expression level of B7H3 in a biological sample from a patient to modify a treatment regimen for the patient, wherein when the expression level of B7H3 is medium or high, the treatment regimen is modified to comprise a B7H3-targeted therapy.

In certain embodiments, the detection comprises immunohistochemical detection, immunofluorescence detection, flow cytometry detection, enzyme-linked immunosorbent detection, protein/peptide microarray detection, immunoblot detection, bead-based immunoassay, and/or microfluidic immunoassay.

In certain embodiments, the patient is a patient with a tumor or cancer.

In certain embodiments, the patient is a patient with a B7H3-related tumor or cancer.

In certain embodiments, the cancer or tumor comprises: bladder cancer, breast cancer, cholangiocarcinoma, colorectal cancer, lymphoma, esophageal cancer, brain glioma, neuroblastoma, head and neck squamous cell carcinoma, kidney cancer, liver cancer, lung cancer, ovarian cancer, pancreatic cancer, prostate cancer, sarcoma, melanoma, gastric cancer, thymus cancer, endometrial cancer, and/or cervical cancer.

Other aspects and advantages of the present application will be readily apparent to those skilled in the art from the following detailed description. Only exemplary embodiments of the present application are shown and described in the following detailed description. As will be recognized by those skilled in the art, the content of the present application enables those skilled in the art to make changes to the specific embodiments disclosed without departing from the spirit and scope of the invention to which the present application relates. Accordingly, the drawings and descriptions in the specification of the present application are only illustrative rather than restrictive.

### BRIEF DESCRIPTION OF THE DRAWINGS

The specific features of the invention to which the present application relates are set forth in the appended claims. The features and advantages of the invention to which the present application relates can be better understood with reference to the exemplary embodiments and drawings described in detail below. The drawings are briefly described below:
FIG. 1 shows immunohistochemistry results of antibodies suitable for B7H3 immunohistochemical detection obtained by screening in B7H3-positive cell lines and B7H3-negative cell lines.
FIG. 2 shows an SDS-PAGE image of the purified B7H3 antibody of the present application. After Coomassie Brilliant Blue staining, the loading amount per lane is 3 µg.
FIG. 3 shows determination of affinity between the B7H3 antibody of the present application and the B7H3 antigen by ELISA, and calculated EC50.
FIG. 4A shows the effective concentration for immunohistochemistry (IHC) of the B7H3 antibody of the present application (cell line data).
FIG. 4B shows the effective concentration for immunohistochemistry (IHC) of the B7H3 antibody (clinical tissue data).
FIG. 4C shows the optimal concentration for immunohistochemistry (IHC) experiments of the B7H3 antibody.
FIG. 5 shows results of detecting the sensitivity of the B7H3 antibody of the present application using brain glioma as an example.
FIG. 6 shows that the B7H3 antibody of the present application has specificity for detecting B7H3 protein.
FIG. 7 shows comparative results of immunohistochemical staining performance between the B7H3 antibody of the present application and a commercial B7H3 antibody in B7H3-positive and B7H3-negative cell lines.
FIG. 8 shows comparative results of immunohistochemical staining performance between the B7H3 antibody of the present application and a commercial B7H3 antibody in clinical samples.
FIG. 9 shows that the B7H3 antibody of the present application has the ability to detect B7H3 protein in multiple tumors.
FIG. 10A shows that the B7H3 antibody of the present application is capable of accurately detecting B7H3 proteins with different expression levels in brain glioma samples.
FIG. 10B shows staining results of the B7H3 antibody described herein and a negative control IgG antibody in negative and positive cell line quality control slides.

### DETAILED DESCRIPTION

The embodiments of the invention of the present application are described below with reference to specific examples, and those skilled in the art can easily understand other advantages and effects of the invention of the present application from the disclosure of this specification.

### Definitions of Terms

In the present application, the terms "B7H3" and "B7-H3" are used interchangeably. B7H3 is also referred to as CD276, and generally refers to B7H3 and the gene encoding the same. B7H3 belongs to the B7 immune co-stimulatory and co-inhibitory family and is a type I transmembrane protein. B7H3 has two isoforms in humans, 2Ig-B7-H3 and 4Ig-B7H3, and one isoform in mice, 2Ig-B7-H3. Human 2Ig-B7-H3 shares 88% amino acid identity with murine 2Ig-B7-H3. B7-H3 has an immunosuppressive function and can reduce the release of type I interferon (IFN) by T cells and decrease the cytotoxicity of NK cells. "B7H3" includes intact B7H3 and fragments thereof, and also includes functional variants, isoforms, species homologs, derivatives, and analogs of B7H3. In the present application, the B7H3 may include human B7H3. For example, human B7H3 may have a Uniprot accession number of Q5ZPR3.

In the present application, the term "isolated" generally refers to a substance obtained by artificial means from its natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

In the present application, the term "isolated antigen-binding protein" generally refers to a protein having antigen-binding ability that has been removed from its naturally occurring state. The "isolated antigen-binding protein" may comprise an antigen-binding moiety, and optionally, a framework or scaffold moiety that allows the antigen-binding moiety to adopt a conformation that facilitates binding of the antigen-binding moiety to an antigen. The antigen-binding protein may comprise, for example, antibody-derived protein framework regions (FRs) or alternative protein framework regions or artificial framework regions with grafted CDRs or CDR derivatives. Such frameworks include, but are not limited to, antibody-derived framework regions comprising mutations introduced, for example, to stabilize the three-dimensional structure of the antigen-binding protein, as well as fully synthetic framework regions comprising, for example, biocompatible polymers. See, e.g., Korndorfer et al., 2003, Proteins: Structure, Function, and Bioinformatics, 53(1): 121-129 (2003); and Roque et al., Biotechnol. Prog. 20: 639-654 (2004). The antigen-binding protein may comprise an antibody. Examples of the antigen-binding protein include, but are not limited to: a rabbit antibody, a human antibody, a humanized antibody, a chimeric antibody, a recombinant antibody, a single-chain antibody, a bifunctional antibody, a trifunctional antibody, a tetrafunctional antibody, a Fab, a Fab', an Fv fragment, an F(ab')₂, an F(ab)₂, an scFv, a di-scFv, a dAb, an IgD antibody, an IgE antibody, an IgM antibody, an IgG1 antibody, an IgG2 antibody, an IgG3 antibody, or an IgG4 antibody and a fragment thereof.

In the present application, the term "CDR", also known as "complementarity-determining region", generally refers to a region in an antibody variable domain, which is highly variable in sequence and/or forms a structurally defined loop. Generally, an antibody comprises six CDRs; three in the VH (HCDR1, HCDR2, HCDR3) and three in the VL (LCDR1, LCDR2, LCDR3). In certain embodiments, a naturally occurring camelid antibody consisting of only heavy chains is also capable of functioning normally and stably in the absence of light chains. The CDRs of the antibody can be determined by a variety of numbering systems, such as CCG, Kabat, AbM, Chothia, IMGT, and a combination of Kabat/Chothia. These numbering systems are known in the art.

In the present application, the terms "variable domain" and "variable region" are used interchangeably and generally refer to a portion of an antibody heavy and/or light chain. The variable domains of the heavy and light chains can be referred to as "V_{H}" and "V_{L}", respectively (or "VH" and "VL", respectively). These domains are generally the most variable portions of an antibody (relative to other antibodies of the same type) and comprise the antigen-binding sites.

In the present application, the term "variable" generally means that certain segments of the variable domains may differ greatly in sequence among antibodies. The variable domain mediates antigen binding and defines the specificity of a specific antibody for its specific antigen. However, the variability is not evenly distributed throughout the entire variable domain. It is generally concentrated in three segments called hypervariable regions (CDRs or HVRs) in the light and heavy chain variable domains. The more highly conserved portions of the variable domains are referred to as framework regions (FRs). The variable domains of natural heavy and light chains each comprise four FRs largely in a β-sheet configuration. The FRs are connected by three CDRs to form a loop connection, and in some cases to form part of a β-sheet structure. The CDRs in each chain are held closely together by the FRs, and, together with the CDRs from the other chain, contribute to the formation of the antigen-binding sites of the antibody (see Kabat et al., Sequences of Immunological Interest, Fifth Edition, National Institute of Health, Bethesda, Md. (1991)).

In the present application, the term "antibody" generally refers to an immunoglobulin or a fragment thereof or a derivative thereof, and encompasses any polypeptide that comprises an antigen-binding site, regardless of whether it is produced *in vitro* or *in vivo*. The term includes, but is not limited to, polyclonal, monoclonal, monospecific, multispecific, nonspecific, humanized, single-chain, chimeric, synthetic, recombinant, hybrid, mutated, and grafted antibodies. Unless otherwise modified by the term "intact", as in "intact antibody", for the purposes of the present disclosure, the term "antibody" also includes antibody fragments such as a Fab, an F(ab')₂, an Fv, an scFv, an Fd, a dAb, and other antibody fragments that retain antigen-binding functions (e.g., specifically binding to B7H3). Generally, such fragments should comprise an antigen-binding domain. The basic 4-chain antibody unit is a heterotetrameric glycoprotein consisting of two identical light chains (LCs) and two identical heavy chains (HCs). An IgM antibody consists of 5 basic heterotetrameric units along with an additional polypeptide called the J chain, and contains 10 antigen-binding sites. In contrast, an IgA antibody comprises 2-5 basic 4-chain units that can polymerize with the J chain to form multivalent combinations. For IgG, the 4-chain unit typically has a molecular weight of about 150,000 daltons. Each L chain is linked to an H chain by one covalent disulfide bond, while the two H chains are linked to each other by one or more disulfide bonds depending on the H chain isotype. Each H and L chain also has regularly spaced intrachain disulfide bonds. Each H chain has a variable domain (VH) at the N-terminus, followed by three (for each of the α and γ chains) constant domains (CH) or four (for the µ and ε isotypes) CH domains. Each L chain has a variable domain (VL) at the N-terminus and a constant domain at its other end. VL corresponds to VH, and CL corresponds to the first constant domain of the heavy chain (CH1). Specific amino acid residues are considered to form an interface between the light and heavy chain variable domains. VH and VL pair together to form a single antigen-binding site. For the structures and properties of different classes of antibodies, see, e.g., Basic and Clinical Immunology, 8th Edition, Daniel P. Sties, Abba I. Terr and Tristram G. Parsolw (eds), Appleton & Lange, Norwalk, Conn., 1994, page 71 and chapter 6. The L chains from any vertebrate species can be divided into one of two distinctly different types, κ and λ, based on the amino acid sequences of constant domains thereof. Immunoglobulins can be divided into different classes or isotypes, based on the amino acid sequences of the constant domains of the heavy chains (CHs). There are currently five classes of immunoglobulins: IgA, IgD, IgE, IgG, and IgM, which have heavy chains designated α, δ, ε, γ, and µ, respectively.

In the present application, the term "antigen-binding fragment" generally refers to one or more fragments having the ability to specifically bind to an antigen (e.g., B7H3). In the present application, the antigen-binding fragment may comprise a Fab, a Fab', an F(ab)₂, an Fv fragment, an F(ab')₂, an scFv, a di-scFv, and/or a dAb.

In the present application, the term "Fab" generally refers to an antigen-binding fragment of an antibody. As described above, an intact antibody may be digested with papain. The antibody is digested with papain to produce two identical antigen-binding fragments, namely "Fab" fragments, and a residual "Fc" fragment (i.e., the Fc region, supra). The Fab fragment may consist of one intact L chain and the variable region of one heavy chain along with the first constant region (C_{H}1) of that H chain (V_{H}).

In the present application, the term "Fab' fragment" generally refers to a monovalent antigen-binding fragment of a human monoclonal antibody, which is slightly larger than the Fab fragment. For example, the Fab' fragment may comprise the entire light chain, the entire heavy chain variable region, and all or part of the first and second constant regions of the heavy chain. For example, the Fab' fragment may also comprise part or all of the 220-330 amino acid residues of the heavy chain.

In the present application, the term "F(ab')2" generally refers to an antibody fragment produced by pepsin digestion of an intact antibody. The F(ab')2 fragment contains two Fab fragments held together by disulfide bonds and part of a hinge region. F(ab')2 fragments have divalent antigen-binding activity and are capable of cross-linking antigens.

In the present application, the term "Fv fragment" generally refers to a monovalent antigen-binding fragment of a human monoclonal antibody, which comprises all or part of the heavy chain variable region and the light chain variable region, and lacks the heavy chain constant region and the light chain constant region. The heavy chain variable region and the light chain variable region comprise, for example, CDRs. For example, an Fv fragment comprises all or part of the amino-terminal variable regions of about 110 amino acids in the heavy and light chains.

In the present application, the term "scFv" generally refers to a fusion protein comprising at least one antibody fragment containing a light chain variable region and at least one antibody fragment containing a heavy chain variable region, wherein the light and heavy chain variable regions are contiguously linked (e.g., via a synthetic linker such as a short flexible polypeptide linker), and are capable of being expressed as a single-chain polypeptide, with the scFv retaining the specificity of the intact antibody from which it is derived. Unless otherwise specified, the scFv, as used herein, may have the VL and VH variable regions in any order (e.g., relative to the N-terminus and C-terminus of the polypeptide), and may comprise VL-linker-VH or VH-linker-VL.

In the present application, the term "dAb" generally refers to an antigen-binding fragment having a VH domain and a VL domain, or having a VH domain or a VL domain. See, e.g., Ward et al. (Nature, 1989 Oct 12; 341(6242): 544-6); Holt et al., Trends Biotechnol., 2003, 21(11): 484-490; and WO 06/030220, WO 06/003388, and other published patent applications by Domantis Ltd. The term "dAb" generally comprises sdAb. The term "sdAb" generally refers to a single-domain antibody. A single-domain antibody generally refers to an antibody fragment consisting only of the variable region of an antibody heavy chain (VH domain) or the variable region of an antibody light chain (VL).

In the present application, the term "VHH" generally relates to variable antigen-binding domains of heavy-chain antibodies from Camelidae (camel, dromedary, llama, alpaca, and the like) (see Nguyen V.K. et al., 2000, The EMBO Journal, 19, 921-930; Muyldermans S., 2001, J Biotechnol., 74, 277-302 and a review of Vanl and schoot P. et al., 2011, Antiviral Research 92, 389-407). A VHH may also be referred to as nanobody (Nb).

In the present application, the term "monoclonal antibody" generally refers to an antibody molecule preparation of a single-molecule composition. Monoclonal antibodies generally have high specificity for a single antigenic site. Moreover, unlike conventional polyclonal antibody formulations (which typically have different antibodies directed against different determinants), each monoclonal antibody is directed against a single determinant on the antigen. In addition to their specificity, the monoclonal antibodies are advantageous in that they can be synthesized by hybridoma culture without contamination by other immunoglobulins. The modifier "monoclonal" indicates the characteristic of an antibody obtained from a population of substantially homogeneous antibodies, and is not to be construed as requiring production of the antibody by any particular method. For example, the monoclonal antibodies used in the present application may be prepared in hybridoma cells, or may be prepared by the recombinant DNA method.

In the present application, the term "chimeric antibody" generally refers to an antibody in which the variable region is derived from one species and the constant region is derived from another species. Generally, the variable region is derived from an antibody ("parent antibody") of a laboratory animal, and the constant region is derived from a human antibody, such that the resulting chimeric antibody is less likely to induce an adverse immune response in a human individual as compared with the parent (e.g., rabbit-derived) antibody.

In the present application, the term "humanized antibody" generally refers to an antibody in which some or all of the amino acids outside the CDRs of a non-human antibody (e.g., a rabbit antibody) are replaced with corresponding amino acids derived from a human immunoglobulin. In the CDRs, small additions, deletions, insertions, substitutions, or modifications of amino acids may also be permissible, so long as they retain the ability of the antibody to bind to a specific antigen. A humanized antibody may optionally comprise at least a portion of a human immunoglobulin constant region. "Humanized antibody" retains antigen specificity similar to that of the original antibody. "Humanized" forms of non-human (e.g., rabbit) antibodies are chimeric antibodies minimally comprising sequences derived from non-human immunoglobulins. In certain cases, CDR residues of a human immunoglobulin (recipient antibody) may be replaced with CDR residues from a non-human species (donor antibody) (such as alpaca, mouse, rat, rabbit, or non-human primate) having the desired properties, affinity, and/or capabilities. In certain cases, FR residues of a human immunoglobulin may be replaced with corresponding non-human residues. Furthermore, humanized antibodies may comprise amino acid modifications that are not present in the recipient antibody or in the donor antibody. These modifications may be made to further improve the properties of the antibodies, such as binding affinity.

The term "fully human antibody" generally refers to an antibody that comprises only human immunoglobulin protein sequences. A fully human antibody may contain mouse glycochains if it is produced in a mouse, in a mouse cell, or in a hybridoma derived from a mouse cell. Similarly, "mouse antibody" or "rat antibody" refers to an antibody that comprises only mouse or rat immunoglobulin sequences, respectively. Fully human antibodies can be generated in the human body or in transgenic animals with human immunoglobulin germline sequences through phage display or other molecular biology methods. Exemplary techniques that can be used to prepare antibodies are described in U.S. Patents: 6,150,584; 6,458,592; and 6,420,140. Other techniques, such as the use of libraries, are known in the art.

In the present application, the term "labeled" is intended to include direct labeling by conjugating a detectable substance to an antigen-binding protein, as well as indirect labeling by reaction with another reagent that is directly labeled. Examples of indirect labeling include detection of a primary antibody using a fluorescently labeled secondary antibody, such that the primary antibody can be detected using fluorescently labeled streptavidin. Examples of labels include, but are not limited to, the following: radioactive isotopes or radionuclides (e.g., 3H, 14C, 35S, 90Y, 99Tc, 111In, 125I, 131I, 177Lu, 166Ho, or 153Sm); fluorescent labels (e.g., FITC, rhodamine, and lanthanide phosphors); enzyme labels (e.g., horseradish peroxidase, luciferase, and alkaline phosphatase); chemiluminescent markers; biotin groups; predetermined polypeptide epitopes recognized by secondary reporter genes (e.g., leucine zipper pair sequences, binding sites for secondary antibodies, metal-binding domains, and epitope tags); and magnetic agents, such as gadolinium chelates.

In the present application, the terms "polypeptide molecule", "polypeptide", and "peptide" are used interchangeably and generally refer to a polymer of amino acid residues. The term "fusion protein" generally refers to a polypeptide having at least two moieties covalently linked together. For example, the polypeptide molecule may comprise a fusion protein. Each moiety may be a polypeptide having a different property. The property may be a biological property, such as activity *in vitro* or *in vivo*. The property may also be a simple chemical or physical property, such as binding to a target molecule, catalysis of a reaction, and the like. The two moieties may be directly linked by a single peptide bond or by a peptide linker.

In the present application, the term "chimeric antigen receptor (CAR)" generally refers to a fusion protein comprising an extracellular domain capable of binding to an antigen and at least one intracellular domain. The CAR is a core component of chimeric antigen receptor T cells (CAR-T), and may comprise an antigen (e.g., a tumor-specific antigen and/or a tumor-associated antigen)-binding domain, a transmembrane domain, a co-stimulatory domain, and an intracellular signaling domain. In the present application, the CAR may be constructed based on the antigen (e.g., B7H3) specificity of an antibody and an intracellular domain for T cell receptor activation. T cells genetically modified to express the CAR can specifically recognize and eliminate malignant cells expressing the target antigen.

In the present application, the term "nucleic acid molecule" generally refers to a nucleotide, deoxyribonucleotide or ribonucleotide of any length in its isolated form, or an analog thereof isolated from its natural environment or artificially synthesized.

In the present application, the term "vector" generally refers to a nucleic acid delivery vehicle in which a polynucleotide encoding a protein can be inserted to achieve expression of the protein. The vector can be introduced into host cells through transformation, transduction, or transfection, allowing the genetic substance elements it carries to be expressed in the host cells. By way of example, the vector may comprise: plasmids; phagemids; cosmids; artificial chromosomes, such as yeast artificial chromosomes (YACs), bacterial artificial chromosomes (BACs), or P1-derived artificial chromosomes (PACs); phages, such as λ phages or M13 phages; animal viruses; and the like. The types of animal viruses used as vectors may include retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including promoter sequences, transcription initiation sequences, enhancer sequences, selection elements, and reporter genes. In addition, the vector may further comprise a replication initiation site. The vector may further comprise components that assist its entry into cells, such as viral particles, liposomes, or protein shells, but is not limited to these substances.

In the present application, the term "cell" generally refers to a single cell, cell line or cell culture that can be or has been a recipient of a subject's plasmid or vector, which includes the nucleic acid molecule of the present disclosure or the vector of the present disclosure. The cell may comprise progeny of a single cell. Due to natural, accidental, or intentional mutations, the progeny may not necessarily be identical to the original parent cell (in terms of the morphology of the total DNA complement or the genome). The cell may include cells transfected *in vitro* with the vector of the present application. The cells may be bacterial (e.g., *Escherichia coli*) cells, yeast cells, or other eukaryotic cells, such as COS cells, Chinese hamster ovary (CHO) cells, CHO-K1 cells, LNCAP cells, HeLa cells, HEK293 cells, COS-1 cells, or NS0 cells.

In the present application, the term "immunoconjugate" generally refers to a conjugate formed by conjugating (e.g., covalently linking through a linker molecule) another agent (e.g., a chemotherapeutic agent, a radioactive element, a cytostatic agent, or a cytotoxic agent) to the antibody or the antigen-binding fragment thereof, wherein the conjugate can deliver the other agent to a target cell (e.g., a tumor cell) through specific binding of the antibody or the antigen-binding fragment thereof to an antigen on the target cell.

In the present application, the term "pharmaceutical composition" generally refers to a composition for preventing/treating a disease or condition. The pharmaceutical composition may comprise the isolated antigen-binding protein described herein, the nucleic acid molecule described herein, the vector described herein, and/or the cell described herein, and optionally a pharmaceutically acceptable adjuvant. Furthermore, the pharmaceutical composition may further comprise suitable formulations of one or more (pharmaceutically effective) carriers, stabilizers, excipients, diluents, solubilizers, surfactants, emulsifiers, and/or preservatives. The acceptable ingredients of the composition are preferably non-toxic to the recipient at the dose and concentration used. The pharmaceutical composition of the present disclosure comprises, but is not limited to, liquid, frozen, and lyophilized compositions.

In the present application, the term "pharmaceutically acceptable carrier" generally comprises pharmaceutically acceptable carriers, excipients, or stabilizers that are non-toxic to cells or mammals being exposed thereto at the doses and concentrations used. The physiologically acceptable carrier may include, for example, a buffer, an antioxidant, a low-molecular-weight (less than about 10 residues) polypeptide, a protein, a hydrophilic polymer, an amino acid, a monosaccharide, a disaccharide, other carbohydrates, a chelating agent, a sugar alcohol, a salt-forming counterion such as sodium, and/or a nonionic surfactant.

In the present application, the term "kit" generally refers to a combination of reagents and other materials. The kit is expected to comprise reagents such as a buffer, a protein stabilizing reagent, a signal generating system (e.g., a fluorescent signal generating system), an antibody, a control protein, and a test container (e.g., a microtiter plate). The term "kit" is not intended to be limited to a particular combination of reagents and/or other materials. For example, the kit may further comprise instructions for using the reagents. The detection kit may be packaged in any suitable manner and typically has components contained within a single container or, where necessary, within multiple containers, together with an instruction sheet for performing detection. For example, the kit may further comprise reagents and/or materials for quality control, such as negative and/or positive quality control samples, and negative and/or positive control reagents. The kit can be prepared by a variety of methods known in the art. For example, a positive quality control sample may comprise a sample known to express a target protein, and a negative quality control sample may comprise a sample known not to express a target antigen.

In the present application, the term "subject" generally refers to a human or non-human animal, including but not limited to cats, dogs, horses, pigs, cows, sheep, rabbits, mice, rats, and monkeys.

In the present application, the term "prevention and/or treatment" not only includes prevention and/or treatment of a disease, but also generally includes preventing the onset of the disease, slowing or reversing the progression of the disease, preventing or slowing the onset of one or more symptoms associated with the disease, reducing and/or alleviating one or more symptoms associated with the disease, reducing the severity and/or duration of the disease and/or any symptoms associated therewith, and/or preventing further increase in the severity of the disease and/or any symptoms associated therewith, and preventing, reducing, or reversing any physiological damage caused by the disease and any pharmacological effect that is generally beneficial to the treatment of a patient.

In the present application, the term "diagnosis" (and grammatical variations thereof, such as "diagnostic" or "diagnostics") generally refers to the identification or classification of a molecular or pathological state, disease, or condition. For example, diagnosis may include identification or classification of cancer, and may also include identification or classification of cancer patients who may benefit from a particular treatment regimen. For example, "diagnosis" may refer to identification of a specific type of cancer. "Diagnosis" may also refer to classification of a specific cancer subtype, for example, based on histopathological criteria or molecular characteristics (e.g., subtypes characterized by one or a set of biomarkers, or by the expression of a specific gene or a protein encoded thereby). "Diagnosis" may include a step of detection and/or a step of interpretation of detection results. In the present application, the term "prognosis" generally refers to the relative probability that a certain future outcome may occur in a subject.

In the present application, the terms "disease" and "condition" are used interchangeably and refer to any change in the state of the body or certain organs that prevents or disrupts the implementation of a function, and/or causes symptoms such as discomfort, dysfunction, distress, or even death in a person suffering from or in contact with the disease. The disease or condition may also be referred to as distemper, ailing, ailment, malady, disorder, sickness, illness, complaint, inderdisposion, or affectation.

In the present application, the term "tumor" generally refers to all neoplastic cell growth and proliferation, whether malignant or benign, and all pre-cancerous and cancerous cells and tissues. The terms "cancer", "cancerous", "cell proliferative condition", "proliferative condition", and "tumor" are not mutually exclusive when referred to herein. In certain embodiments, a tumor may refer to a mass containing a majority of cancer cells, for example, a mass exhibiting cells having characteristics of any cancer described herein. Examples of tumors may include primary tumors of any of the above types of cancer or metastatic tumors at secondary sites derived from any of the above types of cancer.

In the present application, the term "detection" encompasses quantitative or qualitative detection, as well as all other methods for generally determining an analyte and specifically determining a subject to be detected. For example, methods that merely detect the presence or absence of a B7H3 protein in a sample fall within the scope of the present disclosure, and methods that provide data regarding the amount or concentration of the B7H3 protein in the sample also fall within the scope of the present disclosure. The terms "detection", "determination", and "identification" are used interchangeably herein and are all within the scope of the present disclosure.

In the present application, the terms "expression level", "level of expression", or "level" are used interchangeably and generally refer to the amount of a polynucleotide (e.g., messenger RNA (mRNA)) or an amino acid product or protein in a biological sample. "Expression" generally refers to the process by which information encoded by a gene is converted into a structure present and functional in a cell. The expression level may, for example, be measured/quantified/detected based on the protein or mRNA expressed from the gene.

In the present application, the terms "patient" and "subject under treatment" are used interchangeably and include human and non-human animal subjects under treatment, as well as subjects having a formally diagnosed disease, subjects having a disease not yet formally diagnosed, subjects receiving medical treatment, subjects at risk of developing a disease, and the like. For example, a patient may include a person in need of treatment for cancer or a precancerous condition or lesion.

In the present application, the term "biological sample" encompasses various types of samples obtained from an organism and may be used for diagnostic or monitoring assays. The term encompasses blood and other biological fluid samples, solid tissue samples such as biopsy specimens, and tissue cultures or cells derived therefrom and progeny thereof. The term encompasses samples that have been manipulated in any way after collection (e.g., treated with reagents, solubilized, or enriched for certain components). The term encompasses clinical samples and also includes cells in a cell culture, a cell supernatant, a cell lysate, serum, plasma, a biological fluid, and a tissue sample.

In the present application, the term "targeted therapy" generally refers to a cancer treatment using drugs or other substances that interfere with specific target molecules typically involved in the growth of cancer cells, with minimal damage to normal cells so as to achieve an antitumor effect. In contrast, conventional cytotoxic chemotherapeutic agents target all dividing cells.

In the present application, the term "specifically binding" or "specific" generally refers to a measurable and reproducible interaction, such as binding between a target and an antibody, that may determine the presence of the target in the presence of a heterogeneous population of molecules (including biomolecules). For example, an antibody that specifically binds to a target (which may be an epitope) may be an antibody that binds to this target with greater affinity, avidity and ease, and/or for a longer duration than it binds to other targets. In certain embodiments, the antibody specifically binds to an epitope on a protein, wherein the epitope is conserved in proteins of different species. In certain embodiments, specific binding may include, but is not required to be, exclusive binding.

In the present application, the protein, polypeptide, and/or amino acid sequence involved is also to be understood as comprising at least the following ranges: variants or homologs having the same or similar function as the protein or polypeptide.

In the present application, the variant may be, for example, a protein or a polypeptide obtained by substitution, deletion, or addition of one or more amino acids in the amino acid sequence of the protein and/or the polypeptide (e.g., an antibody or a fragment thereof that specifically binds to B7H3). For example, the functional variants may comprise proteins or polypeptides with amino acid changes by substitutions, deletions, and/or insertions of at least 1 (for example, 1-30, 1-20, or 1-10, for another example, 1, 2, 3, 4, or 5) amino acid. The functional variants may substantially retain the biological properties of the protein or the polypeptide before the changes (e.g., substitutions, deletions, or additions). For example, the functional variants may retain at least 60%, 70%, 80%, 90%, or 100% of the biological activity (e.g., antigen-binding ability) of the protein or the polypeptide before the changes. For example, the substitutions may be conservative.

In the present application, the homolog may be a protein or a polypeptide comprising an amino acid sequence having at least about 85% (e.g., at least about 85%, about 90%, about 91%, about 92%, about 93%, about 94%, about 95%, about 96%, about 97%, about 98%, about 99%, or higher) sequence homology to the amino acid sequence of the protein and/or the polypeptide (e.g., an antibody or a fragment thereof that specifically binds to extracellular antigen epitopes of B7H3).

In the present application, the homology generally refers to similarity, likeness, or association between two or more sequences. "Percent sequence homology" can be calculated by the following steps: comparing two sequences to be aligned in a comparison window; determining the number of positions at which nucleic acid bases (e.g., A, T, C, G, and I) or amino acid residues (e.g., Ala, Pro, Ser, Thr, Gly, Val, Leu, Ile, Phe, Tyr, Trp, Lys, Arg, His, Asp, Glu, Asn, Gln, Cys, and Met) are identical in the two sequences to give the number of matched positions; dividing the number of matched positions by the total number of positions in the comparison window (i.e., the window size); and multiplying the result by 100 to give a percent sequence homology. Alignment for determining the percent sequence homology can be achieved in a variety of ways known in the art, for example, using publicly available computer software such as BLAST, BLAST-2, ALIGN, or Megalign (DNASTAR) software. Those skilled in the art can determine suitable parameters for alignment of the sequences, including any algorithms required to achieve optimal alignment in a full-length sequence range or target sequence region being compared. The homology can also be determined by the following methods: FASTA and BLAST. For the description of the FASTA algorithm, see W. R. Pearson and D. J. Lipman, "Improved Tools for Biological Sequence Comparison" (Proc. Natl. Acad. Sci.), 85: 2444-2448, 1988; and D. J. Lipman and W. R. Pearson, "Rapid and Sensitive Protein Similarity Searches", Science, 227: 1435-1441, 1989. For description of the BLAST algorithm, see S. Altschul, W. Gish, W. Miller, E. W. Myers and D. Lipman, "A Basic Local Alignment Search Tool", Journal of Molecular Biology, 215: 403-410, 1990.

In the present application, the term "comprise" or "comprising" generally means including, containing, or encompassing. In certain cases, the term also means "being" or "consisting of...". In the present application, the term "about" generally means varying by 0.5%-10% above or below the stated value, for example, varying by 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the stated value.

### Detailed Description of the Present Disclosure

### Isolated antigen-binding protein

The CDRs of an antibody, also known as complementarity-determining regions, are part of the variable regions. The amino acid residues in these regions may be in contact with antigens or antigenic epitopes. The CDRs of the antibody can be determined by a variety of numbering systems, such as CCG, Kabat, Chothia, IMGT, AbM, a combination of Kabat/Chothia, and the like. These numbering systems are known in the art and can be referenced in detail at, for example, http://www.bioinf.org.uk/abs/index.html#kabatnum. Those skilled in the art can determine the CDRs using different numbering systems based on the sequence and structure of the antibody. When using different numbering systems, the CDRs may vary. In the present application, the CDR encompasses CDR sequences defined according to any CDR definition method; the CDR also encompasses variants thereof, which comprise one or more amino acid substitutions, deletions, and/or additions, such as 1-30, 1-20, or 1-10 (for another example, 1, 2, 3, 4, 5, 6, 7, 8, or 9) amino acid substitutions, deletions and/or insertions in the amino acid sequence of the CDR; the CDR also encompasses homologs thereof, which may be amino acid sequences having at least about 85% (e.g., at least about 85%, about 90%, about 91%, about 92%, about 93%, about 94%, about 95%, about 96%, about 97%, about 98%, about 99%, or higher) sequence homology to the amino acid sequence of the CDR.

In one aspect, the present application provides an isolated antigen-binding protein which may comprise an HCDR1, an HCDR2, and an HCDR3. In certain cases, the HCDR1 comprises an amino acid sequence set forth in any one of SEQ ID NOs: 1, 7, 12, and 14, the HCDR2 comprises an amino acid sequence set forth in any one of SEQ ID NOs: 2, 8, 13, and 15, and the HCDR3 comprises an amino acid sequence set forth in any one of SEQ ID NOs: 3, 9, and 16. In the present application, the isolated antigen-binding protein may comprise an LCDR1, an LCDR2, and an LCDR3, wherein the LCDR1 may comprise an amino acid sequence set forth in any one of SEQ ID NOs: 4, 10, and 17, the LCDR2 may comprise an amino acid sequence set forth in any one of SEQ ID NOs: 5, 11, and 18, and the LCDR3 may comprise an amino acid sequence set forth in any one of SEQ ID NOs: 6 and 19.

In the present application, the isolated antigen-binding protein may comprise an HCDR1, an HCDR2, and an HCDR3, wherein the HCDR1 may comprise the amino acid sequence set forth in SEQ ID NO: 1, the HCDR2 may comprise the amino acid sequence set forth in SEQ ID NO: 2, and the HCDR3 may comprise the amino acid sequence set forth in SEQ ID NO: 3. In the present application, the isolated antigen-binding protein may comprise an LCDR1, an LCDR2, and an LCDR3, wherein the LCDR1 may comprise the amino acid sequence set forth in SEQ ID NO: 4, the LCDR2 may comprise the amino acid sequence set forth in SEQ ID NO: 5, and the LCDR3 may comprise the amino acid sequence set forth in SEQ ID NO: 6.

In the present application, the isolated antigen-binding protein may comprise an HCDR1, an HCDR2, and an HCDR3, wherein the HCDR1 may comprise the amino acid sequence set forth in SEQ ID NO: 7, the HCDR2 may comprise the amino acid sequence set forth in SEQ ID NO: 8, and the HCDR3 may comprise the amino acid sequence set forth in SEQ ID NO: 9. In the present application, the isolated antigen-binding protein may comprise an LCDR1, an LCDR2, and an LCDR3, wherein the LCDR1 may comprise the amino acid sequence set forth in SEQ ID NO: 10, the LCDR2 may comprise the amino acid sequence set forth in SEQ ID NO: 11, and the LCDR3 may comprise the amino acid sequence set forth in SEQ ID NO: 6.

In the present application, the isolated antigen-binding protein may comprise an HCDR1, an HCDR2, and an HCDR3, wherein the HCDR1 may comprise the amino acid sequence set forth in SEQ ID NO: 12, the HCDR2 may comprise the amino acid sequence set forth in SEQ ID NO: 13, and the HCDR3 may comprise the amino acid sequence set forth in SEQ ID NO: 3. In the present application, the isolated antigen-binding protein may comprise an LCDR1, an LCDR2, and an LCDR3, wherein the LCDR1 may comprise the amino acid sequence set forth in SEQ ID NO: 4, the LCDR2 may comprise the amino acid sequence set forth in SEQ ID NO: 5, and the LCDR3 may comprise the amino acid sequence set forth in SEQ ID NO: 6.

In the present application, the isolated antigen-binding protein may comprise an HCDR1, an HCDR2, and an HCDR3, wherein the HCDR1 may comprise the amino acid sequence set forth in SEQ ID NO: 14, the HCDR2 may comprise the amino acid sequence set forth in SEQ ID NO: 15, and the HCDR3 may comprise the amino acid sequence set forth in SEQ ID NO: 16. In the present application, the isolated antigen-binding protein may comprise an LCDR1, an LCDR2, and an LCDR3, wherein the LCDR1 may comprise the amino acid sequence set forth in SEQ ID NO: 17, the LCDR2 may comprise the amino acid sequence set forth in SEQ ID NO: 18, and the LCDR3 may comprise the amino acid sequence set forth in SEQ ID NO: 19. In the present application, the isolated antigen-binding protein may comprise at least one CDR in the amino acid sequences set forth in SEQ ID NO: 20 and SEQ ID NO: 21, and the CDR may comprise a CDR defined according to any definition method, which is not limited to CDRs defined according to the definition methods in the following table. CDRs defined according to any method fall within the protection scope of the claims of the present application if the sequences are identical to SEQ ID NO: 20 or 21.

For example, the amino acid sequence set forth in SEQ ID NO: 20 is defined according to the methods in Table 1, and the isolated antigen-binding protein of the present application may comprise the CDRs as shown in the following table.

**Table 1. CDR sequences defined according to different methods in the amino acid sequence set forth in SEQ ID NO: 20**

| Method | HCDR1 | HCDR2 | HCDR3 |
|---|---|---|---|
| Kabat | NYAMG (SEQ ID NO: 1) | VIGGSGNTYYARWAKG (SEQ ID NO: 2) | GGADIHGYAYGTLGTL (SEQ ID NO: 3) |
| IMGT | GIDLSNYA (SEQ ID NO: 7) | IGGSGNT (SEQ ID NO: 8) | ARGGADIHGYAYGTLGTL (SEQ ID NO: 9) |
| Chothia | GIDLSNY (SEQ ID NO: 12) | GGSGN (SEQ ID NO: 13) | GGADIHGYAYGTLGTL (SEQ ID NO: 3) |
| Contact | SNYAMG (SEQ ID NO: 14) | YIGVIGGSGNTY (SEQ ID NO: 15) | ARGGADIHGYAYGTLGT (SEQ ID NO: 16) |

For example, the amino acid sequence set forth in SEQ ID NO: 21 is defined according to the methods in Table 2, and the isolated antigen-binding protein of the present application may comprise the CDRs as shown in the following table.

**Table 2. CDR sequences defined according to different methods in the amino acid sequence set forth in SEQ ID NO: 21**

| Method | LCDR1 | LCDR2 | LCDR3 |
|---|---|---|---|
| Kabat | QASQSIGTALA (SEQ ID NO: 4) | YASTLAS (SEQ ID NO: 5) | QSYYYSNNNSDGSA (SEQ ID NO: 6) |
| IMGT | QSIGTA (SEQ ID NO: 10) | YAS (SEQ ID NO: 11) | QSYYYSNNNSDGSA (SEQ ID NO: 6) |
| Chothia | QASQSIGTALA | YASTLAS (SEQ ID NO: 5) | QSYYYSNNNSDGSA |
| | (SEQ ID NO: 4) | | (SEQ ID NO: 6) |
| Contact | GTALAWY (SEQ ID NO: 17) | LLIYYASTLA (SEQ ID NO: 18) | QSYYYSNNNSDGS (SEQ ID NO: 19) |

In the present application, the isolated antigen-binding protein may comprise a heavy chain variable region VH. In certain cases, the VH may comprise the amino acid sequence set forth in SEQ ID NO: 20.

In the present application, the isolated antigen-binding protein may comprise a light chain variable region VL. In certain cases, the VL may comprise the amino acid sequence set forth in SEQ ID NO: 21.

In the present application, the isolated antigen-binding protein may comprise a heavy chain constant region, and the heavy chain constant region may be derived from a human human or rabbit IgG heavy chain constant region. For example, the heavy chain constant region may comprise an IgG heavy chain constant region derived from a rabbit. For example, the heavy chain constant region may comprise the amino acid sequence set forth in SEQ ID NO: 22.

In the present application, the isolated antigen-binding protein may comprise a light chain constant region, and the light chain constant region may be derived from a human or rabbit κ chain or λ chain. For example, the light chain constant region may be derived from a rabbit κ chain. For example, the light chain constant region may comprise the amino acid sequence set forth in SEQ ID NO: 23.

In the present application, the isolated antigen-binding protein may comprise an antibody heavy chain HC, and the HC may comprise the amino acid sequence set forth in SEQ ID NO: 24. In the present application, the HC may be encoded by the nucleotide sequence set forth in SEQ ID NO: 26.

In the present application, the isolated antigen-binding protein may comprise an antibody light chain LC, and the LC may comprise the amino acid sequence set forth in SEQ ID NO: 25. In the present application, the LC may be encoded by the nucleotide sequence set forth in SEQ ID NO: 27.

In the present application, the isolated antigen-binding protein may comprise an antibody or an antigen-binding fragment thereof.

In certain embodiments, the antigen-binding fragment may comprise a Fab, a Fab', an Fv fragment, an F(ab')₂, an F(ab)₂, an scFv, a di-scFv, and/or a dAb. For example, when the antigen-binding protein is an scFv, the heavy and light chain variable regions thereof may comprise a linker peptide therebetween.

In certain embodiments, the antibody may comprise a monoclonal antibody, a rabbit antibody, a chimeric antibody, a humanized antibody, a recombinant antibody, a single-chain antibody, a bifunctional antibody, a trifunctional antibody, and a tetrafunctional antibody. For example, the chimeric antibody may comprise a human, goat, monkey, dog, mouse, or rabbit chimeric antibody.

In certain embodiments, the antigen-binding protein may be an IgG antibody.

In certain embodiments, the antigen-binding protein is labeled. For example, the antigen protein may be directly labeled and/or indirectly labeled. The substance used for labeling may be any substance that can be detected directly or indirectly. For example, the labeling may comprise directly labeling the antigen-binding protein by coupling a detectable substance thereto. For example, the labeling may comprise indirect labeling by reaction with an additional reagent that is directly labeled. For example, the labeling may comprise indirect labeling by using an enzyme, fluorescein, or biotin-labeled secondary antibody.

Furthermore, it should be noted that the isolated antigen-binding protein of the present application may comprise heavy and/or light chain sequences with one or more conservative sequence modifications thereto. The so-called "conservative sequence modification" refers to an amino acid modification that does not significantly affect or alter the binding properties of the antibody. Such conservative modifications comprise amino acid substitutions, additions and deletions. Modifications can be introduced into the isolated antigen-binding protein of the present application by standard techniques known in the art, such as point mutations and PCR-mediated mutations. Conservative amino acid substitutions are those in which an amino acid residue is replaced with an amino acid residue having a similar side chain. Groups of amino acid residues having similar side chains are known in the art. Such groups of amino acid residues comprise amino acids with basic side chains (e.g., lysine, arginine, and histidine), amino acids with acidic side chains (e.g., aspartic acid, and glutamic acid), amino acids with uncharged polar side chains (e.g., glycine, asparagine, glutamine, serine, threonine, tyrosine, cysteine, and tryptophan), amino acids with non-polar side chains (e.g., alanine, valine, leucine, isoleucine, proline, phenylalanine, and methionine), amino acids with β-branched side chains (e.g., threonine, valine, and isoleucine), and amino acids with aromatic side chains (e.g., tyrosine, phenylalanine, tryptophan, and histidine). In certain embodiments, one or more amino acid residues in the CDRs of the isolated antigen-binding protein of the present application can be replaced with other amino acid residues of the same side chain group. Those skilled in the art know that some conservative sequence modifications do not eliminate the antigen-binding activity, as can be seen, for example, in Brummell et al., (1993) Biochem 32:1180-8; de Wildt et al., (1997) Prot. Eng. 10:835-41; Komissarov et al., (1997) J. Biol. Chem. 272:26864-26870; Hall et al., (1992) J. Immunol. 149:1605-12; Kelley and O'Connell (1993) Biochem.32:6862-35; Adib-Conquy et al., (1998) Int. Immunol.10:341-6 and Beers et al., (2000) Clin. Can. Res. 6:2835-43.

The B7H3 antigen-binding protein described herein can be identified, screened, or characterized by various assays known in the art.

For example, the antigen-binding activity of the antigen-binding protein or the fusion protein of the present application can be assayed, for example, by known methods such as enzyme-linked immunosorbent assay (ELISA), immunoblotting (e.g., western blotting), flow cytometry (e.g., FACS), immunohistochemistry, and immunofluorescence.

In the present application, the isolated antigen-binding protein is capable of specifically binding to B7H3. In certain embodiments, the binding of the isolated antigen-binding protein to B7H3 can be assayed by ELISA. For example, the antigen-binding protein described herein may bind to B7H3 with an EC₅₀ value of less than or equal to about 0.010 µg/mL.

### Polypeptide molecule, nucleic acid molecule, vector, cell, immunoconjugate, and pharmaceutical composition

In another aspect, the present application provides a polypeptide molecule, which may comprise the isolated antigen-binding protein described herein.

In certain embodiments, the polypeptide molecule may comprise a fusion protein. In certain embodiments, the polypeptide molecule may be a fusion protein.

In certain embodiments, the polypeptide molecule may comprise two antigen-binding domains and an antibody heavy chain constant region. The first antigen-binding domain may comprise the isolated antigen-binding protein according to any aspect of the present application, and the second antigen-binding domain may comprise the isolated antigen-binding protein according to any aspect of the present application.

In another aspect, the present application provides a chimeric antigen receptor, which may comprise the antigen-binding protein described herein.

In certain embodiments, the chimeric antigen receptor may comprise an extracellular domain capable of binding to an antigen, i.e., an antigen-binding domain and at least one intracellular domain. For example, the antigen-binding domain of the chimeric antigen receptor may comprise the antigen-binding protein described herein. For example, the antigen-binding domain of the chimeric antigen receptor may comprise the amino acid sequence of the antigen-binding protein described herein. In certain embodiments, the chimeric antigen receptor may comprise an antigen-binding domain, a hinge region, a transmembrane domain, an intracellular co-stimulatory signaling domain, and an intracellular signaling domain. In certain embodiments, the chimeric antigen receptor may further comprise a signal peptide.

In another aspect, the present application provides an isolated nucleic acid molecule that can encode the isolated antigen-binding protein described herein. For example, the isolated nucleic acid molecule may be produced or synthesized by the following methods: (i) *in vitro* amplification, such as amplification by polymerase chain reaction (PCR); (ii) cloning and recombination; (iii) purification, such as separation by enzymatic cleavage and gel electrophoresis fractionation; or (iv) synthesis, such as chemical synthesis.

In another aspect, the present application provides a vector, which may comprise the nucleic acid molecules described herein. In addition, the vector may further comprise other genes, such as marker genes that allow selection of the vector in suitable host cells and under suitable conditions. Furthermore, the vector may further comprise expression control elements that allow proper expression of the coding region in a suitable host. Such control elements are well known to those skilled in the art and may comprise, for example, promoters, ribosome binding sites, enhancers, and other control elements for regulating gene transcription or mRNA translation. The vector can be introduced into host cells through transformation, transduction, or transfection, allowing the genetic substance elements it carries to be expressed in the host cells. The vector may comprise, for example, plasmids, cosmids, viruses, phages, or other vectors commonly used in, for example, genetic engineering. For example, the vector is an expression vector. In addition, the vector may further comprise components that assist its entry into cells, such as viral particles, liposomes, or protein shells, but is not limited to these substances.

In another aspect, the present application provides a cell, which may comprise the nucleic acid molecule described herein or the vector described herein. In certain embodiments, each type of or each of the host cells may comprise one or one type of nucleic acid molecule or vector described herein. In certain embodiments, each type of or each of the host cells may comprise multiple (e.g., two or more) or multiple types (e.g., two or more types) of nucleic acid molecules or vectors described herein. For example, the vector described herein may be introduced into the host cell, such as a eukaryotic cell, e.g., a plant-derived cell, a fungal cell, or a yeast cell. In certain embodiments, the cell may be a bacterial cell (e.g., *Escherichia coli*), a yeast cell, or other eukaryotic cells, such as a COS cell, a Chinese hamster ovary (CHO) cell, a CHO-K1 cell, an LNCAP cell, a HeLa cell, a 293T cell, a COS-1 cell, an SP2/0 cell, an NS0 cell, or a myeloma cell. The vector described herein can be introduced into the host cells based on methods known in the art, e.g., electroporation, lipofectine transfection, and lipofectamin transfection.

In certain embodiments, the cell described herein may comprise a cell comprising the antigen-binding protein described herein, the nucleic acid molecule described herein, the vector described herein, and/or the chimeric antigen receptor described herein. In certain embodiments, the cell may comprise an immune cell. For example, the immune cell may comprise a plasma cell, a T cell, a B cell, a natural killer (NK) cell, a natural killer T (NKT) cell, a mast cell, and a myeloid-derived phagocyte. For example, the immune cell may be autologous/autogeneic or non-autologous. For example, the immune cell may be unmodified or modified.

In another aspect, the present application further provides an immunoconjugate, which may comprise the isolated antigen-binding protein described herein.

In certain embodiments, the isolated antigen-binding protein described herein or the fragment thereof may be linked to another agent, such as a chemotherapeutic agent, a toxin, an immunotherapeutic agent, an imaging probe, or a spectroscopic probe. Such linkage may be achieved through one or more covalent bonds or non-covalent interactions, and may comprise chelation. Various linkers (which may be known in the art) may be used to form the immunoconjugate. In addition, the immunoconjugate may be provided in the form of a fusion protein, which may be expressed from a polynucleotide encoding the immunoconjugate. The immunoconjugate may further comprise, for example, an antibody-drug conjugate (ADC). Suitable drugs may comprise cytotoxins, alkylating agents, DNA minor groove binding molecules, DNA intercalators, DNA cross-linking agents, histone deacetylase inhibitors, nuclear export inhibitors, proteasome inhibitors, inhibitors of topoisomerase I or II, heat shock protein inhibitors, tyrosine kinase inhibitors, antibiotics, and antimitotic agents. In ADCs, the antibody and therapeutic agent may be cross-linked by a linker that is cleavable, e.g., a peptide linker, a disulfide linker, or a hydrazone linker.

In another aspect, the present application further provides a pharmaceutical composition, which may comprise the isolated antigen-binding protein described herein, the polypeptide molecule described herein, the chimeric antigen receptor described herein, the immunoconjugate described herein, the nucleic acid molecule described herein, the vector described herein, and/or the cell described herein, and optionally a pharmaceutically acceptable carrier.

In certain embodiments, the pharmaceutical composition may further comprise suitable formulations of one or more (pharmaceutically effective) adjuvants, stabilizers, excipients, diluents, solubilizers, surfactants, emulsifiers, and/or preservatives. The acceptable ingredients of the composition are preferably non-toxic to the recipient at the dose and concentration used. The pharmaceutical composition of the present disclosure comprises, but is not limited to, liquid, frozen, and lyophilized compositions.

In certain embodiments, the pharmaceutical composition may further comprise more than one active compound, typically those having complementary activities that do not adversely affect one another. The type and effective amount of such medicaments may depend, for example, on the amount and type of antagonists present in the formulation, as well as on the clinical parameters of the subject.

In certain embodiments, the pharmaceutically acceptable carrier may comprise any and all solvents, dispersion media, coatings, isotonic agents, and absorption delaying agents, which are compatible with pharmaceutical administration and are generally safe and non-toxic.

In certain embodiments, the pharmaceutical composition may be administered parenterally, transdermally, intracavitarily, intra-arterially, intrathecally, and/or intranasally, or by direct injection into tissue. For example, the pharmaceutical composition may be administered to a patient or subject by infusion or injection. In certain embodiments, administration of the pharmaceutical composition may be performed by different routes, such as intravenous, intraperitoneal, subcutaneous, intramuscular, topical, or intradermal administration. In certain embodiments, the pharmaceutical composition may be administered without interruption. Such uninterrupted (or continuous) administration may be achieved by a small pump system worn by the patient to measure the flow of therapeutic agent into the patient, as described in WO2015/036583.

In another aspect, the present application further provides a reagent, which may comprise the isolated antigen-binding protein described herein, the polypeptide molecule described herein, the chimeric antigen receptor described herein, the immunoconjugate described herein, the nucleic acid molecule described herein, the vector described herein, and/or the cell described herein. The reagent described herein may comprise any substance for use in analyzing biological samples, without being limited by the state of the substance or by the distribution of the substance alone or in combination with other substances.

In another aspect, the present application provides an assay plate, which may comprise the isolated antigen-binding protein described herein, the polypeptide molecule described herein, the chimeric antigen receptor described herein, the immunoconjugate described herein, the nucleic acid molecule described herein, the vector described herein, and/or the cell described herein. In certain embodiments, the assay plate described herein may comprise a component or container for use in supporting and a reagent for use in detection, and the reagent for use in detection may comprise the isolated antigen-binding protein described herein, the polypeptide molecule described herein, the chimeric antigen receptor described herein, the immunoconjugate described herein, the nucleic acid molecule described herein, the vector described herein, and/or the cell described herein. For example, the reagent may be a test strip comprising the antigen-binding protein described herein. For example, the test strip may comprise an antigen-coated region.

In another aspect, the present application provides a kit, which may comprise the isolated antigen-binding protein described herein, the polypeptide molecule described herein, the chimeric antigen receptor described herein, the immunoconjugate described herein, the nucleic acid molecule described herein, the vector described herein, and/or the cell described herein.

In certain embodiments, the kit described herein may comprise the isolated antigen-binding protein described herein.

In certain embodiments, the kit described herein may comprise the isolated antigen-binding protein described herein, and the isolated antigen-binding protein is capable of specifically binding to B7H3 and comprises HCDR1, HCDR2, and HCDR3 as defined in any one of the following groups (a) to (d):
a) the HCDR1 comprising the amino acid sequence set forth in SEQ ID NO: 1, the HCDR2 comprising the amino acid sequence set forth in SEQ ID NO: 2, and the HCDR3 comprising the amino acid sequence set forth in SEQ ID NO: 3;
b) the HCDR1 comprising the amino acid sequence set forth in SEQ ID NO: 7, the HCDR2 comprising the amino acid sequence set forth in SEQ ID NO: 8, and the HCDR3 comprising the amino acid sequence set forth in SEQ ID NO: 9;
c) the HCDR1 comprising the amino acid sequence set forth in SEQ ID NO: 12, the HCDR2 comprising the amino acid sequence set forth in SEQ ID NO: 13, and the HCDR3 comprising the amino acid sequence set forth in SEQ ID NO: 3; or
d) the HCDR1 comprising the amino acid sequence set forth in SEQ ID NO: 14, the HCDR2 comprising the amino acid sequence set forth in SEQ ID NO: 15, and the HCDR3 comprising the amino acid sequence set forth in SEQ ID NO: 16.

In certain embodiments, the isolated antigen-binding protein comprised in the kit described herein may further comprise LCDR1, LCDR2, and LCDR3 as defined in any one of the following groups (a) to (c):
a) the LCDR1 comprising the amino acid sequence set forth in SEQ ID NO: 4, the LCDR2 comprising the amino acid sequence set forth in SEQ ID NO: 5, and the LCDR3 comprising the amino acid sequence set forth in SEQ ID NO: 6;
b) the LCDR1 comprising the amino acid sequence set forth in SEQ ID NO: 10, the LCDR2 comprising the amino acid sequence set forth in SEQ ID NO: 11, and the LCDR3 comprising the amino acid sequence set forth in SEQ ID NO: 6; or
c) the LCDR1 comprising the amino acid sequence set forth in SEQ ID NO: 17, the LCDR2 comprising the amino acid sequence set forth in SEQ ID NO: 18, and the LCDR3 comprising the amino acid sequence set forth in SEQ ID NO: 19.

In certain embodiments, the isolated antigen-binding protein comprised in the kit described herein may comprise a heavy chain variable region (VH), and the VH comprises the amino acid sequence set forth in SEQ ID NO: 20. In certain embodiments, the isolated antigen-binding protein comprised in the kit described herein may comprise a light chain variable region (VL), and the VL comprises the amino acid sequence set forth in SEQ ID NO: 21. In certain embodiments, the isolated antigen-binding protein comprised in the kit described herein may comprise a heavy chain variable region having the amino acid sequence set forth in SEQ ID NO: 20 and a light chain variable region having the amino acid sequence set forth in SEQ ID NO: 21.

In certain embodiments, the kit described herein may comprise the isolated antigen-binding protein described herein, and the concentration of the antigen-binding protein is adjusted based on actual usage. For example, the concentration of the isolated antigen-binding protein in the kit may be greater than or equal to 0.10 µg/mL. For example, the concentration of the isolated antigen-binding protein may be greater than or equal to 0.10 µg/mL, greater than or equal to 0.15 µg/mL, greater than or equal to 0.20 µg/mL, greater than or equal to 0.25 µg/mL, greater than or equal to 0.30 µg/mL, greater than or equal to 0.35 µg/mL, greater than or equal to 0.40 µg/mL, greater than or equal to 0.45 µg/mL, greater than or equal to 0.50 µg/mL, greater than or equal to 0.60 µg/mL, greater than or equal to 0.70 µg/mL, greater than or equal to 0.80 µg/mL, greater than or equal to 0.90 µg/mL, greater than or equal to 1.0 µg/mL, greater than or equal to 1.5 µg/mL, greater than or equal to 2.0 µg/mL, greater than or equal to 2.5 µg/mL, greater than or equal to 3.0 µg/mL, greater than or equal to 3.5 µg/mL, greater than or equal to 4.0 µg/mL, greater than or equal to 4.5 µg/mL, greater than or equal to 5.0 µg/mL, greater than or equal to 5.5 µg/mL, greater than or equal to 6.0 µg/mL, greater than or equal to 6.5 µg/mL, greater than or equal to 7.0 µg/mL, greater than or equal to 7.5 µg/mL, greater than or equal to 8.0 µg/mL, greater than or equal to 8.5 µg/mL, greater than or equal to 9.0 µg/mL, greater than or equal to 10.0 µg/mL, or higher. For example, the concentration of the isolated antigen-binding protein in the kit may be less than or equal to 10.0 µg/mL. For example, the concentration of the isolated antigen-binding protein may be less than or equal to 9.5 µg/mL, less than or equal to 9.0 µg/mL, less than or equal to 8.5 µg/mL, less than or equal to 8.0 µg/mL, less than or equal to 7.5 µg/mL, less than or equal to 7.0 µg/mL, less than or equal to 6.5 µg/mL, less than or equal to 6.0 µg/mL, less than or equal to 5.5 µg/mL, less than or equal to 5.0 µg/mL, less than or equal to 4.5 µg/mL, less than or equal to 4.0 µg/mL, less than or equal to 3.5 µg/mL, less than or equal to 3.0 µg/mL, less than or equal to 2.5 µg/mL, less than or equal to 2.0 µg/mL, less than or equal to 1.5 µg/mL, less than or equal to 1.0 µg/mL, less than or equal to 0.9 µg/mL, less than or equal to 0.8 µg/mL, less than or equal to 0.7 µg/mL, less than or equal to 0.6 µg/mL, less than or equal to 0.5 µg/mL, less than or equal to 0.4 µg/mL, less than or equal to 0.3 µg/mL, less than or equal to 0.2 µg/mL, less than or equal to 0.1 µg/mL, or lower. For example, the concentration of the isolated antigen-binding protein in the kit may be at 0.1 µg/mL-10 µg/mL. For example, the concentration of the isolated antigen-binding protein may be 10 µg/mL, 3.5 µg/mL, 1 µg/mL, 0.7 µg/mL, or 0.5 µg/mL.

In certain embodiments, the kit can be used in a variety of detection assays, including but not limited to, immunohistochemical detection, enzyme-linked immunosorbent detection, protein/peptide microarray detection, immunoblot detection, bead-based immunoassay, and/or microfluidic immunoassay.

In certain embodiments, the kit may further comprise a secondary antibody that specifically recognizes the antigen-binding protein of the present application. In certain embodiments, the secondary antibody further comprises a detectable label. In certain embodiments, the secondary antibody may comprise a rabbit antibody.

In certain embodiments, the kit may further comprise a reagent and/or material for use in quality control. For example, the reagent and/or material for use in quality control may comprise a negative quality control sample, a negative quality control material, a positive quality control sample, and/or a positive quality control material. For example, the material for use in quality control may comprise a cell line section. For example, the traceability of the cell line section for use in quality control is clear. For example, the negative quality control material may comprise a stably passaged human cell line that does not express B7H3. For example, the positive quality control material may comprise a section of a stably passaged human cell line expressing B7H3 at a medium or high level. In certain embodiments, the kit may further comprise a reagent and/or material for use as a control. For example, the reagent and/or material for use as a control may comprise a negative control reagent. For example, the negative control reagent may comprise an IgG isotype antibody.

The kit of the present application may further comprise other materials required for the kit. In certain embodiments, the kit may comprise additional additives, such as stabilizers, buffers (e.g., a blocking buffer or a lysis buffer), and the like. For example, the reagent in the kit may be provided as a dry powder, which is typically lyophilized, including an excipient, which, upon reconstitution, provides a reagent solution at an appropriate concentration. For example, the other material may comprise an antibody dilution reagent. For example, the antibody dilution reagent may comprise a protein stabilizer, a bacteriostatic agent, and a preservative. For example, the antibody dilution reagent may comprise a phosphate buffered saline. For example, the antibody dilution reagent may comprise, but is not limited to, one or more of the following components: phosphate buffered saline (PBS), bovine serum albumin (BSA), proclin 300, 0.05% sodium azide, Tris-HCl buffered saline solution, sucrose, and Triton x-100. For example, the antibody dilution reagent may comprise 0.01 M PBS (phosphate buffered saline) containing 1% bovine serum albumin (BSA) and 0.05% proclin 300.

In certain embodiments, the kit may comprise an immunohistochemical detection kit. For example, the immunohistochemical kit may comprise the antigen-binding protein described herein and other materials for use in immunohistochemical detection. For example, the immunohistochemical kit may comprise a reagent for antigen retrieval, a reagent for blocking, a chromogenic reagent, a chromogenic enhancement reagent, and/or a signal enhancement reagent. For example, the other materials for use in immunohistochemical detection are known to those skilled in the art.

In certain embodiments, the reagent for antigen retrieval may comprise a heat-induced antigen retrieval buffer and/or an enzymatic antigen retrieval reagent. For example, the reagent for antigen retrieval may comprise, but is not limited to, a sodium citrate buffer, an EDTA antigen retrieval solution, and/or a Tris-EDTA buffer. For example, the sodium citrate buffer may comprise 10 mM sodium citrate and 0.05% Tween-20, with a pH of 6.0. For example, the EDTA antigen retrieval solution may comprise 1 mM EDTA with a pH of 8.0. For example, the Tris-EDTA buffer may comprise 10 mM Tris, 1 mM EDTA solution, and 0.05% Tween-20, with a pH of 9.0.

In certain embodiments, the reagent for blocking may comprise, but is not limited to, a hydrogen peroxide blocking reagent, IHC blocking serum, a protein blocking buffer, and/or a biotin blocking agent. For example, the IHC blocking serum may comprise goat serum. For example, the protein blocking buffer may comprise skim milk powder.

In certain embodiments, the chromogenic reagent may comprise a substrate and an enzyme that converts the substrate into a chromogenic product. For example, the chromogenic reagent may comprise, but is not limited to, 3,3'-diaminobenzidine (DAB), 3-amino-9-ethylcarbazole (AEC), p-nitrophenyl phosphate (p-NPP), horseradish peroxidase (HRP), and alkaline phosphatase (AP).

In certain embodiments, the chromogenic enhancement reagent may comprise a horseradish peroxidase (HRP) polymer and an alkaline phosphatase (AP) polymer.

In certain embodiments, the signal enhancement reagent comprises an enzyme-labeled secondary antibody that specifically recognizes the isolated antigen-binding protein. For example, the signal enhancement reagent may comprise a horseradish peroxidase-labeled secondary antibody. For example, the secondary antibody may comprise a rabbit antibody.

The components of the kit of the present application may be pre-attached to a solid support, or may be applied to the surface of a solid support when the kit is used. In certain embodiments, the kit may be used in combination with other reagents and/or devices to obtain detection results.

The components of the kit described herein may be packaged together or separately in two or more containers. In certain embodiments, the container may be a vial containing a sterile lyophilized formulation of a composition suitable for reconstitution.

In certain embodiments, the kit may further comprise instructions for use.

### Preparation method

In another aspect, the present application provides a method for preparing the isolated antigen-binding protein. The method may comprise culturing the cell described herein under conditions that allow the expression of the antigen-binding protein, for example, by adopting an appropriate culture medium, an appropriate temperature, and an appropriate incubation time. These methods are known to those of ordinary skill in the art.

Any method suitable for producing monoclonal antibodies can be used to produce the antigen-binding protein of the present application. For example, an animal may be immunized with linked or naturally occurring B7H3 or a fragment thereof. Suitable immunization protocols can be employed, comprising the use of adjuvants, immunostimulants, and repeated booster immunizations, and one or more routes of administration may be used. In certain embodiments, isolated antigen-binding proteins targeting B7H3 can be obtained by extracting peripheral blood lymphocytes from immunized rabbits, extracting nucleic acid fragments from the cells and cloning the nucleic acid fragments into a vector, and screening and enriching the isolated antigen-binding proteins targeting B7H3 using a phage surface display system.

B7H3 in any suitable form can be used as an immunogen (antigen) for generating a non-human antibodies specific for B7H3 and for screening the biological activity of the antibodies. For example, the stimulating immunogen may be full-length B7H3, including a native homodimer, or a peptide containing a single epitope/multiple epitopes. The immunogen may be used alone or in combination with one or more immunogenicity-enhancing agents known in the art.

The method described herein may further comprise a step of isolating the antigen-binding protein. For example, the method may comprise isolating the antigen-binding protein from a culture.

### Method and use

In another aspect, the present application provides a method for detecting B7H3 in a sample, which comprises administering the isolated antigen-binding protein described herein, the polypeptide molecule described herein, the chimeric antigen receptor described herein, the immunoconjugate described herein, the nucleic acid molecule described herein, the vector described herein, the cell described herein, the pharmaceutical composition described herein, the reagent described herein, the assay plate described herein, and/or the kit described herein. In another aspect, the present application provides use of the isolated antigen-binding protein described herein, the polypeptide molecule described herein, the chimeric antigen receptor described herein, the immunoconjugate described herein, the nucleic acid molecule described herein, the vector described herein, the cell described herein, the pharmaceutical composition described herein, the reagent described herein, the assay plate described herein, and/or the kit described herein in preparing a B7H3 detection reagent.

In another aspect, the present application provides use of the isolated antigen-binding protein described herein, the polypeptide molecule described herein, the chimeric antigen receptor described herein, the immunoconjugate described herein, the nucleic acid molecule described herein, the vector described herein, the cell described herein, the pharmaceutical composition described herein, the reagent described herein, the assay plate described herein, and/or the kit described herein in detecting B7H3.

In certain embodiments, the method may comprise:
(a) providing a sample from a subject;
(b) directly or indirectly contacting the isolated antigen-binding protein described herein, the polypeptide molecule described herein, the chimeric antigen receptor described herein, the immunoconjugate described herein, the nucleic acid molecule described herein, the vector described herein, the cell described herein, the pharmaceutical composition described herein, the reagent described herein, the assay plate described herein, and/or the kit described herein with the sample;
(c) determining the level of B7H3 in the sample; and/or
(d) comparing the level of B7H3 in the sample to one or more reference values.

In certain embodiments, the sample may comprise a biological sample and/or a non-biological sample. For example, the biological sample may comprise a clinical sample. For example, the biological sample may comprise cells in a cell culture, a cell supernatant, a cell lysate, serum, plasma, a biological fluid, and/or a tissue sample. For example, the biological sample may be a formalin-fixed paraffin-embedded tissue section. For example, the biological sample may comprise a tissue microarray. In certain embodiments, the method may further comprise a step of processing the sample such that it is suitable for detection.

In certain embodiments, detecting B7H3 in the sample may comprise qualitative and/or quantitative detection of B7H3 in the sample. For example, the detection may comprise qualitative and/or quantitative detection of B7H3 protein in the sample. For example, the B7H3 may comprise an endogenous B7H3 protein. For example, the B7H3 protein may comprise an endogenous B7H3 protein in tumor cells.

In certain embodiments, detecting B7H3 in the sample may comprise detecting the expression level of B7H3 in the sample. In certain embodiments, detecting B7H3 in the sample may comprise qualitative and/or quantitative detection of cells expressing B7H3 in the sample. In certain embodiments, detecting B7H3 in the sample may comprise detecting the distribution of cells expressing B7H3 in the sample. For example, the cells expressing B7H3 may comprise tumor cells.

The detection method described herein may comprise detecting B7H3 by any method of administering the isolated antigen-binding protein described herein, the polypeptide molecule described herein, the chimeric antigen receptor described herein, the immunoconjugate described herein, the nucleic acid molecule described herein, the vector described herein, the cell described herein, the pharmaceutical composition described herein, the reagent described herein, the assay plate described herein, and/or the kit described herein. For example, the method may comprise, but is not limited to, immunohistochemical detection, enzyme-linked immunosorbent detection, protein/peptide microarray detection, immunoblot detection, bead-based immunoassay, and/or microfluidic immunoassay.

In certain embodiments, the method may comprise detecting B7H3 in a sample by immunohistochemistry. For example, the method may comprise administering the isolated antigen-binding protein described herein to detect B7H3 in a sample.

In certain embodiments, the method for detecting B7H3 in a sample may comprise the following steps:
a) contacting the sample with a labeled isolated antigen-binding protein of the present application; and
b) for the labeled antibody bound to B7H3 in the sample, performing detection by measuring fluorescence, chemiluminescence, or radioactivity based on the type of label bound to the labeled antibody.

The method for detecting B7H3 described herein may have one or more of the following advantages: good detection sensitivity; good detection specificity; capable of binding to most commercially available rabbit secondary antibodies and enabling detection in various ways; and capable of directly visually observing the presence or absence and the intensity of B7H3 by immunostaining and enabling semi-quantitatively or qualitatively analysis of the expression of B7H3 protein, with a simple detection procedure.

In certain embodiments, the reference value may be the level of B7H3 in one or more control samples. For example, the one or more control samples may comprise (i) one or more samples obtained from normal healthy individuals; (ii) one or more samples obtained from patients with a disease not related to B7H3; (iii) one or more samples obtained from tissues or organs of patients with a B7H3-related disease that are not affected by the B7H3-related disease; and/or (iv) a combination thereof.

In certain cases, the method for detecting B7H3 in a sample is for non-therapeutic purposes. In certain cases, the method for detecting B7H3 in a sample is not a diagnostic method.

In another aspect, the present application provides a method for preventing and/or treating a disease and/or condition, which comprises administering the isolated antigen-binding protein described herein, the polypeptide molecule described herein, the chimeric antigen receptor described herein, the immunoconjugate described herein, the nucleic acid molecule described herein, the vector described herein, the cell described herein, the pharmaceutical composition described herein, the reagent described herein, the assay plate described herein, and/or the kit described herein.

In another aspect, the present application provides use of the isolated antigen-binding protein described herein, the polypeptide molecule described herein, the chimeric antigen receptor described herein, the immunoconjugate described herein, the nucleic acid molecule described herein, the vector described herein, the cell described herein, the pharmaceutical composition described herein, the reagent described herein, the assay plate described herein, and/or the kit described herein in preventing and/or treating a disease and/or condition.

In another aspect, the present application provides use of the isolated antigen-binding protein described herein, the polypeptide molecule described herein, the chimeric antigen receptor described herein, the immunoconjugate described herein, the nucleic acid molecule described herein, the vector described herein, the cell described herein, the pharmaceutical composition described herein, the reagent described herein, the assay plate described herein, and/or the kit described herein in preparing a medicament for use in preventing and/or treating a disease and/or condition.

In the present application, the administration may be performed in different ways, such as intravenous, intratumoral, intraperitoneal, subcutaneous, intramuscular, topical, or intradermal administration.

In another aspect, the present application provides a method for diagnosing a disease and/or condition, which comprises administering the isolated antigen-binding protein described herein, the polypeptide molecule described herein, the chimeric antigen receptor described herein, the immunoconjugate described herein, the nucleic acid molecule described herein, the vector described herein, the cell described herein, the pharmaceutical composition described herein, the reagent described herein, the assay plate described herein, and/or the kit described herein. The method for diagnosing a disease and/or condition described herein may comprise qualitatively and/or quantitatively detecting B7H3 in a sample using the method for detecting B7H3 described herein. In certain embodiments, the presence of a disease may be confirmed, the formation or risk of formation of a disease may be assessed, the progression and/or prognosis of a disease may be evaluated, and/or a population responsive to a treatment can be screened based on the qualitative and/or quantitative detection results of B7H3 in the sample. In another aspect, the present application provides use of the isolated antigen-binding protein described herein, the polypeptide molecule described herein, the chimeric antigen receptor described herein, the immunoconjugate described herein, the nucleic acid molecule described herein, the vector described herein, the cell described herein, the pharmaceutical composition described herein, the reagent described herein, the assay plate described herein, and/or the kit described herein in preparing a detection reagent.

In another aspect, the present application provides use of the isolated antigen-binding protein described herein, the polypeptide molecule described herein, the chimeric antigen receptor described herein, the immunoconjugate described herein, the nucleic acid molecule described herein, the vector described herein, the cell described herein, the pharmaceutical composition described herein, the reagent described herein, the assay plate described herein, and/or the kit described herein in diagnosing a disease and/or condition.

In certain embodiments, the detection reagent may comprise a product for detection and/or diagnosis in any form, without limitation as to the form in which it exists. For example, the detection reagent may comprise an *in vitro* diagnostic reagent. The diagnostic reagent described herein may comprise products such as reagents, kits, calibrators, and quality control products for *in vitro* detection of human samples in processes including disease prediction, prevention, diagnosis, treatment monitoring, prognosis evaluation, and health status assessment, and may be used alone or in combination with instruments, apparatuses, devices, or systems.

In certain embodiments, diagnosing the disease and/or condition may comprise, or the detection reagent may be used for:
a) detecting B7H3 in a sample;
b) detecting endogenous B7H3 protein in tumor cells;
c) detecting tumor cells expressing B7H3; or
d) detecting expression level of B7H3 in a biological sample from a patient to predict or determine prognosis and/or progression of a disease and/or condition;
e) detecting expression level of B7H3 in a biological sample from a patient to determine a patient population for administration of a B7H3-targeted therapy, wherein when the expression level of B7H3 is medium or high, the B7H3-targeted therapy is administered to the patient;
f) detecting expression level of B7H3 in a biological sample from a patient to predict or determine efficacy of a B7H3-targeted therapy administered to the patient, wherein when the expression level of B7H3 is medium or high, it indicates that the B7H3-targeted therapy is therapeutically effective; and/or
g) detecting expression level of B7H3 in a biological sample from a patient to modify a treatment regimen for the patient, wherein when the expression level of B7H3 is medium or high, the treatment regimen is modified to comprise a B7H3-targeted therapy.

In the present application, detection and/or diagnostic results can be interpreted using various methods and standards known in the art. In certain embodiments, the severity of the disease and/or condition described herein is positively correlated with the level of B7H3. For example, the detection and/or diagnostic results can be interpreted based on consensus diagnostic guidelines in the art. For example, when detection is performed by immunohistochemical staining, interpretation may be carried out based on the percentage-based grading of positive cells, the staining intensity of positive cells, or a combination of both the percentage-based grading of positive cells and the staining intensity of positive cells. For example, the staining intensity may be positively correlated with the level of B7H3. For example, the interpretation of immunohistochemical staining results may be performed using a semi-quantitative scoring system. For example, when diaminobenzidine (DAB) is used as the chromogenic reagent, the grading of B7H3 staining intensity may be as follows: a score of 0 indicates no staining; a score of 1 indicates light yellow or weak staining; a score of 2 indicates brown-yellow or moderate staining; and a score of 3 indicates brown or strong staining. For example, the density of B7H3-positive cells may be scored as follows: a score of 0 indicates no stained cells; a score of 1 indicates 1%-10% of cells stained; a score of 2 indicates 11%-50% of cells stained; and a score of 3 indicates >50% of cells stained. The final interpretation of staining results in pathological tissues is obtained by multiplying the staining intensity score and the positive cell density score and then combining the results, wherein a score of 0 indicates negative expression, a score of 1 indicates weak positive expression (+), a score of 2-3 indicates moderate positive expression (++), and a score greater than 3 indicates strong positive expression (+++). In addition to the above interpretation indexes for the staining results, other interpretation indexes may also be derived from the staining intensity and the staining percentage based on actual needs.

In certain embodiments, the medium or high expression level of B7H3 is relative to one or more reference values. In certain embodiments, the reference value may be the level of B7H3 in one or more control samples. For example, the one or more control samples may comprise (i) one or more samples obtained from normal healthy individuals; (ii) one or more samples obtained from patients with a disease not related to B7H3; (iii) one or more samples obtained from tissues or organs of patients with a B7H3-related disease that are not affected by the B7H3-related disease; and/or (iv) a combination thereof.

In the present application, the disease and/or condition may comprise a B7H3-related disease and/or condition. For example, the disease and/or condition may comprise a disease and/or condition associated with B7H3 expression. For example, the disease and/or condition may comprise a tumor and/or cancer associated with B7H3 expression. For example, the disease and/or condition may comprise a disease and/or condition with a medium or high expression level of B7H3. For example, the disease and/or condition described herein may comprise, but is not limited to: bladder cancer, breast cancer, cholangiocarcinoma, colorectal cancer, lymphoma, esophageal cancer, brain glioma, head and neck squamous cell carcinoma, kidney cancer, liver cancer, lung cancer, ovarian cancer, pancreatic cancer, prostate cancer, sarcoma, melanoma, gastric cancer, thymus cancer, endometrial cancer, and/or cervical cancer.

In the present application, the patient is a patient with a B7H3-related disease and/or condition. For example, the patient is a patient with a disease and/or condition associated with abnormal expression of B7H3. In certain embodiments, the patient may comprise a patient with a tumor and/or cancer. For example, the patient may comprise, but is not limited to, a patient with bladder cancer, breast cancer, cholangiocarcinoma, colorectal cancer, lymphoma, esophageal cancer, brain glioma, head and neck squamous cell carcinoma, kidney cancer, liver cancer, lung cancer, ovarian cancer, pancreatic cancer, prostate cancer, sarcoma, melanoma, gastric cancer, thymus cancer, endometrial cancer, and/or cervical cancer.

The isolated antigen-binding protein described herein, the polypeptide molecule described herein, the chimeric antigen receptor described herein, the immunoconjugate described herein, the nucleic acid molecule described herein, the vector described herein, the cell described herein, the pharmaceutical composition described herein, the reagent described herein, the assay plate described herein, and/or the kit described herein can be used for immunohistochemical detection of tumor tissues, thereby improving the accuracy of immunohistochemical detection of tumor specimens, facilitating the diagnosis of malignant tumors and determination of the primary site and pathological classification, and improving the diagnostic accuracy of tumors, particularly poorly differentiated or undifferentiated tumors. The present application further comprises the following embodiments:
1. A kit, comprising an isolated antigen-binding protein capable of specifically binding to B7H3, wherein the isolated antigen-binding protein comprises HCDR1, HCDR2, and HCDR3 as defined in any one of the following groups (a) to (d):
   a) the HCDR1 comprising the amino acid sequence set forth in SEQ ID NO: 1, the HCDR2 comprising the amino acid sequence set forth in SEQ ID NO: 2, and the HCDR3 comprising the amino acid sequence set forth in SEQ ID NO: 3;
   b) the HCDR1 comprising the amino acid sequence set forth in SEQ ID NO: 7, the HCDR2 comprising the amino acid sequence set forth in SEQ ID NO: 8, and the HCDR3 comprising the amino acid sequence set forth in SEQ ID NO: 9;
   c) the HCDR1 comprising the amino acid sequence set forth in SEQ ID NO: 12, the HCDR2 comprising the amino acid sequence set forth in SEQ ID NO: 13, and the HCDR3 comprising the amino acid sequence set forth in SEQ ID NO: 3; or
   d) the HCDR1 comprising the amino acid sequence set forth in SEQ ID NO: 14, the HCDR2 comprising the amino acid sequence set forth in SEQ ID NO: 15, and the HCDR3 comprising the amino acid sequence set forth in SEQ ID NO: 16.
2. The kit according to embodiment 1, wherein the isolated antigen-binding protein may further comprise LCDR1, LCDR2, and LCDR3 as defined in any one of the following groups (a) to (c):
   a) the LCDR1 comprising the amino acid sequence set forth in SEQ ID NO: 4, the LCDR2 comprising the amino acid sequence set forth in SEQ ID NO: 5, and the LCDR3 comprising the amino acid sequence set forth in SEQ ID NO: 6;
   b) the LCDR1 comprising the amino acid sequence set forth in SEQ ID NO: 10, the LCDR2 comprising the amino acid sequence set forth in SEQ ID NO: 11, and the LCDR3 comprising the amino acid sequence set forth in SEQ ID NO: 6; or
   c) the LCDR1 comprising the amino acid sequence set forth in SEQ ID NO: 17, the LCDR2 comprising the amino acid sequence set forth in SEQ ID NO: 18, and the LCDR3 comprising the amino acid sequence set forth in SEQ ID NO: 19.
3. The kit according to any one of embodiments 1 and 2, wherein the isolated antigen-binding protein comprises a heavy chain variable region (VH), and the VH comprises the amino acid sequence set forth in SEQ ID NO: 20.
4. The kit according to any one of embodiments 1-3, wherein the isolated antigen-binding protein comprises a light chain variable region (VL), and the VL comprises the amino acid sequence set forth in SEQ ID NO: 21.
5. The kit according to any one of embodiments 1-4, wherein the isolated antigen-binding protein comprises a heavy chain variable region having the amino acid sequence set forth in SEQ ID NO: 20 and a light chain variable region having the amino acid sequence set forth in SEQ ID NO: 21.
6. The kit according to any one of embodiments 1-5, wherein the isolated antigen-binding protein has a concentration of 0.1 µg/mL-10 µg/mL.
7. The kit according to any one of embodiments 1-6, wherein the kit comprises a reagent and/or material for use in quality control.
8. The kit according to any one of embodiments 1-7, wherein the kit comprises a negative quality control sample and/or a positive quality control sample.
9. The kit according to embodiment 8, wherein the negative quality control sample comprises a section of a human cell line that does not express B7H3.
10. The kit according to any one of embodiments 8 and 9, wherein the positive quality control sample comprises a section of a human cell line expressing B7H3 at a medium or high level.
11. The kit according to any one of embodiments 1-10, wherein the kit comprises a kit/material for use as a control.
12. The kit according to any one of embodiments 1-11, wherein the kit comprises a negative control reagent.
13. The kit according to any one of embodiments 1-13, wherein the kit comprises a negative control reagent, and the negative control reagent comprises an IgG isotype antibody.
14. The kit according to any one of embodiments 1-13, wherein the kit comprises an antibody dilution reagent.
15. The kit according to embodiment 14, wherein the antibody dilution reagent comprises a protein stabilizer, a bacteriostatic agent, and a preservative.
16. The kit according to any one of embodiments 14-15, wherein the antibody dilution reagent may comprise a phosphate buffered saline.
17. The kit according to embodiment 16, wherein the phosphate buffered saline may comprise 0.01 M phosphate buffered saline (PBS) containing 1% bovine serum albumin (BSA) and 0.05% proclin 300.
18. The kit according to any one of embodiments 1-17, wherein the kit is an immunohistochemical detection kit, an enzyme-linked immunosorbent detection kit, a protein/peptide microarray detection kit, an immunoblot detection kit, a bead-based immunoassay kit, or a microfluidic immunoassay kit.
18. The kit according to any one of embodiments 1-17, wherein the kit is an immunohistochemical detection kit and the kit further comprises a reagent for antigen retrieval, a reagent for blocking, a chromogenic reagent, a chromogenic enhancement reagent, and/or a signal enhancement reagent.
19. The kit according to embodiment 18, wherein the reagent for antigen retrieval may comprise a heat-induced antigen retrieval buffer and/or an enzymatic antigen retrieval reagent.
20. The kit according to any one of embodiments 18 and 19, wherein the reagent for antigen retrieval may comprise a sodium citrate buffer, an EDTA antigen retrieval solution, and/or a Tris-EDTA buffer.
21. The kit according to embodiment 20, wherein the sodium citrate buffer may comprise 10 mM sodium citrate and 0.05% Tween-20, with a pH of 6.0; the EDTA antigen retrieval solution may comprise 1 mM EDTA with a pH of 8.0; and the Tris-EDTA buffer may comprise 10 mM Tris, 1 mM EDTA solution, and 0.05% Tween-20, with a pH of 9.0.
22. The kit according to any one of embodiments 18-21, wherein the reagent for blocking comprises a hydrogen peroxide blocking reagent, IHC blocking serum, a protein blocking buffer, and a biotin blocking agent.
23. The kit according to embodiment 22, wherein the IHC blocking serum may comprise goat serum.
24. The kit according to embodiment 22, wherein the protein blocking buffer may comprise skim milk powder.
25. The kit according to any one of embodiments 18-24, wherein the chromogenic reagent comprises a substrate and an enzyme that converts the substrate into a chromogenic product.
26. The kit according to any one of embodiments 18-25, wherein the chromogenic reagent comprises 3,3'-diaminobenzidine (DAB), 3-amino-9-ethylcarbazole (AEC), p-nitrophenyl phosphate (p-NPP), horseradish peroxidase (HRP), and/or alkaline phosphatase (AP).
27. The kit according to any one of embodiments 18-26, wherein the chromogenic enhancement reagent comprises a horseradish peroxidase (HRP) polymer and/or an alkaline phosphatase (AP) polymer.
28. The kit according to any one of embodiments 18-27, wherein the signal enhancement reagent comprises an enzyme-labeled secondary antibody that specifically recognizes the isolated antigen-binding protein.
29. The kit according to any one of embodiments 18-28, wherein the signal enhancement reagent may comprise a horseradish peroxidase-labeled secondary antibody.
30. The kit according to any one of embodiments 28 and 29, wherein the secondary antibody comprises a rabbit antibody.
31. A method for detecting B7H3, comprising using the kit according to any one of embodiments 1-29.
32. The method according to embodiment 31, wherein the method comprises:
   a) providing a sample from a subject;
   b) contacting the kit directly or indirectly with the sample;
   c) determining B7H3 level in the sample; and/or
   d) comparing the B7H3 level in the sample to one or more reference values.
33. Use of the kit according to any one of embodiments 1-29, wherein the kit is used for:
   a) detecting B7H3 in a sample;
   b) detecting endogenous B7H3 protein in tumor cells;
   c) detecting tumor cells expressing B7H3;
   d) detecting expression level of B7H3 in a biological sample from a patient to predict or determine prognosis and/or progression of a disease and/or condition;
   e) detecting expression level of B7H3 in a biological sample from a patient to determine a patient population for administration of a B7H3-targeted therapy, wherein when the expression level of B7H3 is medium or high, the B7H3-targeted therapy is administered to the patient;
   f) detecting expression level of B7H3 in a biological sample from a patient to predict or determine efficacy of a B7H3-targeted therapy administered to the patient, wherein when the expression level of B7H3 is medium or high, it indicates that the B7H3-targeted therapy is therapeutically effective; and/or
   g) detecting expression level of B7H3 in a biological sample from a patient to modify a treatment regimen for the patient, wherein when the expression level of B7H3 is medium or high, the treatment regimen is modified to comprise a B7H3-targeted therapy.
34. The use according to embodiment 33, wherein the patient is a patient with a tumor or cancer.
35. The use according to any one of embodiments 33 and 34, wherein the patient is a patient with a B7H3-related tumor or cancer.
36. The use according to any one of embodiments 33-35, wherein the cancer or tumor comprises: bladder cancer, breast cancer, cholangiocarcinoma, colorectal cancer, lymphoma, esophageal cancer, brain glioma, neuroblastoma, head and neck squamous cell carcinoma, kidney cancer, liver cancer, lung cancer, ovarian cancer, pancreatic cancer, prostate cancer, sarcoma, melanoma, gastric cancer, thymus cancer, endometrial cancer, and/or cervical cancer. Without being bound by any theory, the following examples are intended only to illustrate the antigen-binding protein, preparation method, use, and the like, of the present application, and are not intended to limit the scope of the invention of the present application.

### Examples

The experimental instruments used in the examples of the present application are shown in the following table:

| Name and model | | | | |
|---|---|---|---|---|
| Name | Model | | | |
| Horizontal drying oven | | OV75WN | | |
| Desktop microwave oven | | M1-L213B white 00003657 | | |
| Decolorizing shaker | | TS-1000 | | |
| Microscope | | ECLIPSE Ei | | |
| The experimental reagents used in the examples of the present application are shown in the following table: | | | | |

| List of reagents | | | | |
|---|---|---|---|---|
| Reagent name | Cat. No. | Supplier | Host | Antibody type |
| B7H3 primary antibody (antibody 36H7) | | | Rabbit | Primary antibody |
| Rabbit (DA1E) mAb IgG XPIsotype Control | 3900S | CST | Rabbit | |
| Rabbit two-step detection kit (rabbit enhanced polymer method detection system) | PV-9001 | Beijing ZSGB-BIO | | Secondary antibody |
| Citrate buffered saline | ZLI-9065 | Beijing ZSGB-BIO | | |
| PBS | ZIL-9062 | Beijing ZSGB-BIO | | |
| TBST | T1087 | Solarbio | | |
| Peroxidase blocking solution | ZIL-9310 | Beijing ZSGB-BIO | | |
| Goat serum | ZIL-9022 | Beijing ZSGB-BIO | | |
| BSA | 9048-46-8 | Sangon | | |
| DAB chromogenic kit | ZIL-9017 | Beijing ZSGB-BIO | | |
| Hematoxylin | 0500-0100 | Zhuo Ding Biological | | |
| Differentiation solution | 0500-0190 | Zhuo Biological | Ding | |
| Bluing solution | 0500-0130 | Zhuo Biological | Ding | |
| Neutral balsam | ZIL-9516 | Beijing ZSGB-BBIO | | |

### Preparation of reagents:

1% BSA: When preparing 5 mL of solution, 0.05 g of BSA was weighed out and added to 5 mL of PBS, and the mixture was well mixed by shaking and used when no bubbles were present. DAB chromogenic reagent: The volume ratio of the DAB substrate solution (reagent 2) to the concentrated DAB solution (reagent 1) was 1 mL:50 µL, and the required volume was calculated based on the number of samples.

### Immunohistochemistry experiment:

The methods used in the immunohistochemistry experiment of the present disclosure all follow the following immunohistochemistry experiment steps.

### Deparaffinization/hydration:

1) The sections were incubated at 60 °C for 60 min;
2) the sections were immersed in xylene for 3 times, each for 5 min;
3) the sections were immersed in 100% ethanol for 2 times, each for 5 min;
4) the sections were immersed in 70% ethanol for 2 times, each for 5 min; and
5) the sections were washed with water for 2 times, each for 5 min.

### Antigen retrieval:

1) Microwave retrieval: The sections were immersed in 1× citrate retrieval solution, heated to boiling by microwave, maintained in a sub-boiling state (95 °C-98 °C) for 20 min, and allowed to cool naturally for 30 min.

### Staining:

1) The sections were washed with water for 3 times, each for 5 min;
2) the sections were covered with 3% hydrogen peroxide for 10 min;
3) the sections were washed with water for 2 times, each for 5 min;
4) the sections were washed with TBST for 2 times, each for 5 min;
5) 150 µL of blocking solution was added, and the mixture was blocked at room temperature for 10 min;
6) the blocking solution was removed, 150 µL of primary antibody (diluted to the recommended concentration) was added, and the mixture was incubated at 37 °C for 1 h;
7) the sections were washed with TBST for 2 times, each for 5 min;
8) 150 µL of reaction enhancer (ZSGB-BIO) was added, and the resulting mixture was left to stand in a humidified chamber at 25 °C for 20 min;
9) the sections were washed with TBST for 2 times, each for 5 min;
10) 150 µL of secondary antibody (ZSGB-BIO) was added, and the resulting mixture was left to stand in a humidified chamber at 25 °C for 30min;
11) the sections were washed with TBST for 2 times, each for 5 min;
12) a DAB chromogenic reagent was prepared according to the instructions;
13) 150 µL of the prepared DAB chromogenic reagent was added for chromogenic reaction for 2 min, and the redundant chromogenic reagent was removed by washing with flowing water;
14) the sections were stained with hematoxylin for 5 min, incubated with a differentiation solution for 15 s, and washed with water for 3 min;
15) the sections were stained with a bluing solution for 15 s and washed with water for 3 min;
16) the sections were immersed in 100% ethanol for 5 min, and then immersed in xylene for 2-3 times, each for 5 min; and
the sections were mounted with neutral balsam and observed under a microscope.

### Example 1. Screening and Recombinant Production of B7H3 Antibody

In this example, rabbits were immunized with a B7H3 extracellular polypeptide, rabbit monoclonal antibodies specifically recognizing B7H3 protein were screened, and recombinant expression was performed by molecular cloning. Gene sequences of rabbit monoclonal antibodies specifically recognizing B7H3 protein were constructed into plasmids and transfected into cells to obtain cell lines expressing the rabbit monoclonal antibodies specifically recognizing B7H3 protein. The cells were expanded, the culture solution was collected, and the B7H3 antibody of the present application was obtained after purification. The specific experiment was as follows:

### 1. Screening of B7H3 rabbit monoclonal antibody

A recombinant human B7-H3 protein antigen fused with mouse C-terminal Fc and a 6×His tag was purchased from Wuhan DIMA Biotechnology Co., Ltd. The reference sequence was derived from the B7H3 protein sequence with Swiss-Prot accession number Q5ZPR3-2. The B7H3 immunogen covered the amino acid sequence spanning residues 29-245 of the extracellular domain of the B7H3 protein. Rabbits were immunized three times with the B7H3 protein antigen. Serum of the immunized rabbits was collected, and the B7H3 antibody titer in the serum after three immunizations was determined by indirect ELISA. Rabbits with an OD value greater than 2.7 at a serum dilution of 1:256,000 were selected. Blood was collected from the rabbits, and B7H3 antibody-positive B cells were enriched by flow cytometry. Single B cells were cultured, and primary screening was performed using a non-target protein mFc-His tag protein. The culture supernatants of the screened B cells were subjected to ELISA re-screening using B7H3-mFc-His as the coating antigen. 293 cells overexpressing B7H3 protein were used for flow cytometry analysis of the supernatants obtained after the above screening, and candidate positive clones were identified (Table 1).

**Table 1**

| | Clone No. | | | | | | | | | | |
|---|---|---|---|---|---|---|---|---|---|---|---|
| Batch 1 | 34D4 | 34H2 | 36A1 | 36H7 | 38A4 | 38H3 | 39C8 | 39D6 | 39B11 | 39C9 | 37B6 |
| Batch 2 | 32A2 | 38F4 | 37E6 | 32F1 | 36A11 | 35A8 | 37A7 | 36D7 | 34H5 | 38F8 | 39H3 |

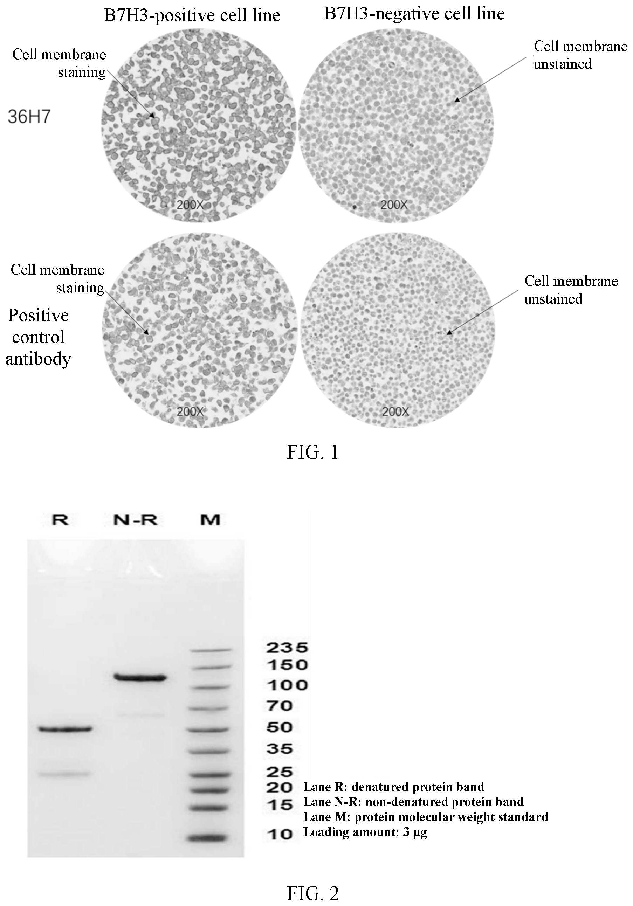

Plasmids were constructed for the above positive clones and transfected into 293 cells for small-scale expression of B7H3 antibodies. The antibodies were purified using Protein A. Immunohistochemistry was performed using FFPE sections of B7H3-positive cell lines to screen positive antibody clones. Finally, a rabbit monoclonal antibody 36H7 suitable for immunohistochemistry was selected (FIG. 1).

### 2. Production of B7H3 rabbit monoclonal antibody

### (1) Synthesis of B7H3 antibody gene DNA sequence

The DNA sequences of the heavy chain and light chain of the B7H3 antibody (clone No. 36H7) were synthesized. Specific primers were used to amplify the synthesized sequences, and sufficient amounts of the heavy chain and light chain DNA sequences of the 36H7 antibody were obtained for subsequent recombination.

### (2) Plasmid recombination

The antibody DNA fragment plasmids were recombined according to the reaction system in the following table to construct expression plasmids:

| Gel extraction of target PCR products | Linearized vector | Recombinase | Reaction condition |
|---|---|---|---|
| 4 µL | 3.5 µL | 2.5 µL | Placing in a water bath at 50 °C for 25 min, allowing to stand for 2-3 min to lower the temperature, performing transformation and bacterial solution coating experiments, and incubating at 37 °C overnight |

### (3) Colony screening experiment

1) The constructed plasmids were transfected into *Escherichia coli*, and the bacteria were cultured overnight. Single colonies were picked from the plates after overnight incubation.
2) Colony PCR was performed using specific primers, and the PCR amplification products were subjected to electrophoresis to identify positive clones. The sequences of the specific primers were as follows:
   BI-CMV-F: CGCAAATGGGCGGTAGGCGTG (SEQ ID NO: 28);
   BI-SEQ-R: AGCGTAAAAGGAGCAACATAGT (SEQ ID NO: 29).
3) 4 positive colonies were randomly selected and cultured. DNA was extracted from the cultured positive bacterial solutions and amplified using primers, and the amplified products were sequenced for identification.
4) Positive clone bacterial solutions with correct sequencing results were selected, low-endotoxin plasmids were extracted after expansion culture, and sequencing verification was performed.

### (4) Plasmid extraction

A commercial plasmid extraction kit was used to extract plasmids. The concentration and purity of the plasmids were determined, and sufficient plasmids were obtained.

### (5) Transfection and expression (taking 20 mL as an example)

1) 145 M cells were collected and centrifuged to remove the supernatant.
2) About 0.5 mL of electroporation buffer was added to the cells, and after the mixture was well mixed, 2 mg-3 mg of a mixed solution of heavy and light chain plasmids (at a ratio of 1:1, at a concentration of 500 ng/µL) was separately added.
3) The above cell-plasmid suspension was thoroughly mixed, and 1 mL of the mixture was transferred into a 1 mL electroporation cuvette. The cuvette was placed into an electroporator for electroporation.
4) After the electroporation was completed, the cells in the cuvette were transferred into a shake flask containing 20 mL of pre-prepared culture solution and incubated statically for 40 min.
5) After the incubation was completed, the shake flask was cultured at 37 °C and 270 rpm with 8% CO₂. After 24 h, feed/sodium butyrate/dual antibiotics were added, and the culture was performed for another 3-7 days. Samples were collected on day 5 to determine the concentration of the B7H3 (36H7) antibody.

### (6) Antibody purification: The antibody was purified using a Protein A affinity chromatography column:

1) Chromatography column equilibrium: 1× PBS, flow rate of 1 mL/min, 20 mL
2) Loading: flow rate of 1 mL/min
3) Washing: 1× PBS, flow rate of 1 mL/min, 20 mL
4) Elution: sodium acetate buffer (pH 3.4), 1 mL/min, collected in separate tubes, about 500 µL in each tube. A total of 10 tubes were collected, and the absorbance at 280 nm was measured using a NanoDrop instrument.
5) Dialysis: The high-concentration protein was pipetted into a dialysis bag and dialyzed against 1× PBS in a beaker.
6) An antibody with a purity of not less than 95% was obtained.

### 3. Quality control of B7H3 rabbit monoclonal antibody

### (1) Purity detection (SEC-HPLC)

Acceptance criterion: antibody purity ≥95%.

An LC-20AT high-performance liquid chromatograph and a gel chromatography column were used to perform SEC analysis under the following conditions:

| Parameter | Value |
|---|---|
| Flow rate | 1 ml/min |
| Injection volume | Concentration ≤ 4 mg/mL:20 µL |
| | Concentration > 4 mg/mL:50 µg |
| Column temperature | 35 °C |
| Detection wavelength | 214 nm, 280 nm |
| Collection time | 15 min |

Water was replaced with the mobile phase, and the flow rate was slowly increased to 1.000 mL/min until a stable baseline was achieved. 25 µL of the antibody was transferred into a correspondingly numbered injection vial, and the sample vial was placed at the corresponding position of the instrument, with an injection time of 15 min. The data were analyzed and processed and saved. The mobile phase was replaced with deionized water, and the system was flushed for 1.5 h.

### (2) Endotoxin detection

1) Preparation of test solution:
   Sample dilution factor: MVD = C·L/λ
   *Note: MVD: maximum effective dilution factor of the test sample, L: bacterial endotoxin limit of the test sample (1 EU/mg), C: concentration of the test sample, λ: labeled sensitivity of the Limulus reagent. Sampling volume of 200 µL of test solution = C/MVD × 200 µL
2) Preparation of positive control solution: An aqueous solution containing 2λ endotoxin standard was prepared.
3) Preparation of positive control solution for test sample: A test sample solution containing 2λ endotoxin standard was prepared.
4) Sample loading: 4 vials of Limulus reagent that had passed the sensitivity verification (sensitivity: 0.06 EU/mL) were taken and opened after wiping with 75% alcohol cotton balls. Each vial was reconstituted with 0.1 mL of water for endotoxin testing for subsequent use. Negative control tube: 10 µL of water for endotoxin testing; positive control tube: 100 µL of positive control solution (0.12 EU/mL); sample positive control tube: 100 µL of test sample positive solution; test sample tube: 100 µL of test solution.
5) Reaction: The tube openings were sealed, and the tube was gently shaken to mix well and vertically incubated in a 37 °C thermostatic incubator for 60 min.
6) Acceptance criteria for experimental results.

| Limulus reagent (0.06 EU/mL) | Water for endotoxin testing | Sample | Acceptance criteria for experimental results |
|---|---|---|---|
| Negative control tube | 200ul | None | - |
| Positive control tube | 100ul | 100 µL endotoxin standard (0.12 EU/mL) | + |
| Sample positive control tube | 100ul | 100 µL of sample positive control solution | + |
| Test sample tube | 100ul | 100 µL of test solution | - |

### (3) Molecular weight determination of B7H3 antibody

1) Appropriate concentrations of separating gel and stacking gel were prepared according to the size of the protein.
2) The prepared separating gel was poured into the gel casting plates and overlaid with water. After solidification, the overlay water was removed, the stacking gel was added, and a comb was inserted. After solidification, the comb was removed, and a buffer was added to submerge the gel.
3) Treatment of denatured sample: A certain volume of the sample was pipetted, diluted according to the dilution ratio of the protein loading buffer, and subjected to heat treatment. The sample was centrifuged, and the supernatant was collected for electrophoresis.
4) The denatured samples, non-denatured samples, and protein Marker were sequentially loaded into the wells. The electrophoresis tank was covered, the initial voltage was set to 80 V to start electrophoresis, and after the samples entered the separating gel, the voltage was increased to 100 V until completion of electrophoresis.
5) After the electrophoresis was completed, the gel was taken out and stained with a staining solution. When the gel became opaque, destaining was initiated using a destaining solution until a clear background was obtained, followed by photographing for record.

Results: The theoretical molecular weight of the B7H3 antibody (clone No. 36H7) was 142 kDa, and the molecular weights of the heavy chain and light chain thereof were 48 kDa and 23 kDa, respectively. The protein electrophoresis gel results showed that the molecular weight of the non-denatured B7H3 antibody was 100 kDa-150 kDa, and after denaturation, the molecular weights of the heavy chain and light chain of the B7H3 antibody were about 50 kDa and 25 kDa, respectively, which were consistent with the theoretical molecular weight of the B7H3 antibody, indicating that the produced B7H3 antibody was consistent with the screened B7H3 antibody (FIG. 2).

### Example 2. Affinity Assay of B7H3 Antibody

This example demonstrates that the antibody of the present application is capable of specifically binding to B7H3 and exhibits high affinity. An ELISA method was used to determine the affinity of the antibody described herein.

The experimental procedures were as follows:
(1) Preparation of B7H3 antigen-coated microplate: 100 µL of B7H3 antigen at a concentration of 0.5 µg/mL (Human B7-H3/CD276 Protein, His Tag (MALS verified)) was added to each well of a microplate, and the microplate was incubated at 4 °C for 16 h and then washed 5 times with PBST. Subsequently, 200 µL of 5% BSA was added to each well, and the microplate was incubated at 25 °C for 60 min and then washed 5 times with PBST.
(2) Incubation with primary antibody: B7H3 antibodies (clone No. 36H7) at different concentrations were added, with each concentration tested in duplicate wells, and the microplate was incubated at 25 °C for 1 h and then washed 5 times with PBST.
(3) Incubation with secondary antibody: An HRP-labeled goat anti-rabbit secondary antibody was added, and the microplate was incubated at 25 °C for 0.5 h and then washed 5 times with PBST.
(4) Color development: A chromogenic reagent was added, the microplate was incubated at 25 °C for 15 min, and then a stop reagent was added.
(5) Detection and analysis: Absorbance was measured at a wavelength of 450 nm using a microplate reader. An absorbance curve was plotted. An absorbance curve was obtained, and an EC₅₀ value was calculated.

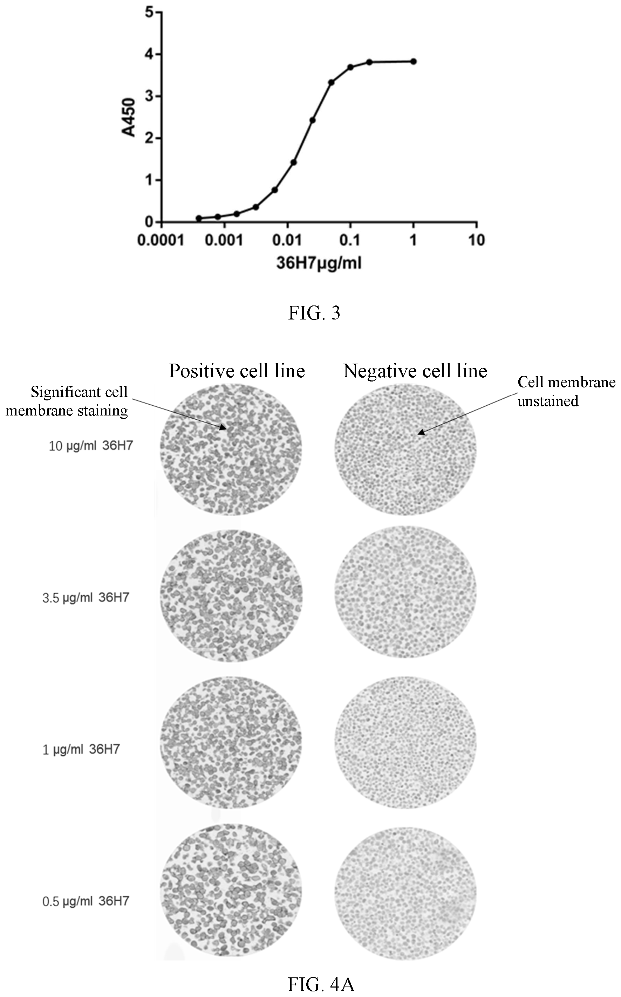

The affinity determination results are shown in Table 2. After four-parameter fitting, the absorbance and the concentration of the B7H3 antibody exhibited an S-shaped curve. When the antibody concentration was ≥ 0.1 µg/mL, the absorbance entered a plateau phase. The EC₅₀ was 0.01807 µg/mL, indicating that the B7H3 antibody of the present application had high affinity for the B7H3 antigen (FIG. 3).

| Table 2. Affinity determination results | | | | | | | | |
|---|---|---|---|---|---|---|---|---|
| Concentrati on µg/mL | 1 | 0.2 | 0.1 | 0.05 | 0.025 | 0.0125 | 0.0062 5 | 0.00312 5 |
| A450 | 3.84 | 3.798 | 3.751 | 3.394 | 2.488 | 1.481 | 0.783 | 0.362 |
| | 3.85 | 3.796 | 3.667 | 3.256 | 2.396 | 1.397 | 0.758 | 0.358 |
| | 3.799 | 3.846 | 3.667 | 3.343 | 2.404 | 1.398 | 0.768 | 0.367 |
| Concentrati on µg/mL | 0.001562 5 | 0.0007812 5 | 0.00039 1 | 0.00019 5 | 9.77E-05 | 4.88E-05 | 2.44E-05 | 1.22E-05 |
| A450 | 0.197 | 0.122 | 0.092 | 0.088 | 0.068 | 0.066 | 0.076 | 0.068 |
| | 0.199 | 0.129 | 0.099 | 0.082 | 0.079 | 0.069 | 0.064 | 0.067 |
| | 0.2 | 0.127 | 0.086 | | 0.067 | | 0.063 | 0.071 |

### Example 3. B7H3 Antibody Exhibiting High Titer

This example demonstrates that the B7H3 antibody of the present application exhibits a high titer, and is capable of detecting B7H3 expression at relatively low concentrations without significant background staining.

In this example, a B7H3-positive cell line and a B7H3-negative cell line were selected to verify the effect of different concentrations of B7H3 antibodies (clone No. 36H7) on B7H3 detection. Four B7H3 antibody concentrations were selected for immunohistochemistry experiment verification, namely 10 µg/mL, 3.5 µg/mL, 1 µg/mL, and 0.5 µg/mL, in descending order. As shown in the immunohistochemistry results of FIG. 4A, at all four concentrations, the B7H3 antibody produced clear positive signals in the B7H3-positive cell line, with obvious cell membrane staining, indicating that the B7H3 antibody was capable of specifically recognizing the extracellular antigen of B7H3. In contrast, no obvious specific staining was observed for the B7H3 antibody in the B7H3-negative cell line, and only slight background staining was observed at high antibody concentrations; however, the background staining of the negative cell line gradually disappeared as the antibody concentration decreased.

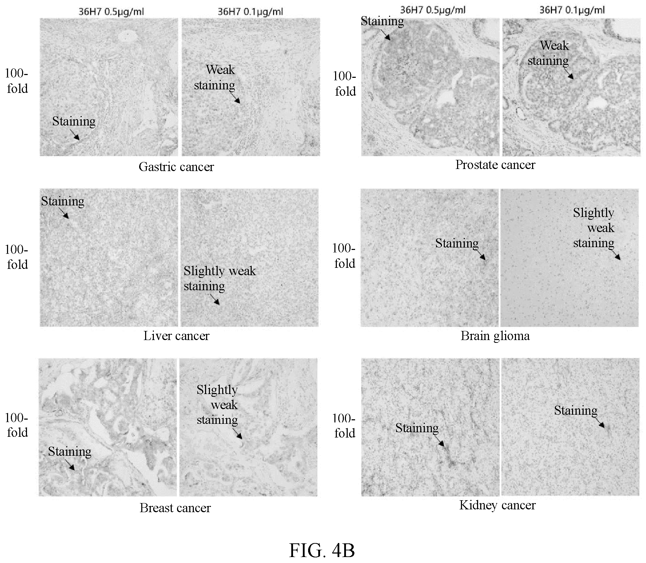

To further investigate the minimum titer of the B7H3 antibody, based on the above cell line experiments, B7H3-positive gastric cancer, prostate cancer, liver cancer, and brain glioma samples were selected for further verification. The staining performance of the B7H3 antibody at concentrations of 0.5 µg/mL and 0.1 µg/mL was verified, respectively. As shown in the immunohistochemistry results of FIG. 4B, at an antibody concentration of 0.5 µg/mL, clear staining signals were observed in all the B7H3-positive gastric cancer, prostate cancer, liver cancer, and brain glioma samples. At an antibody concentration of 0.1 µg/mL, weaker staining signals were observed in the B7H3-positive gastric cancer, prostate cancer, liver cancer, and brain glioma samples, indicating that the immunohistochemical staining signal increased correspondingly with increasing B7H3 antibody concentration. When the B7H3 antibody concentration was ≥ 0.5 µg/mL, a satisfactory staining effect was obtained in immunohistochemistry.

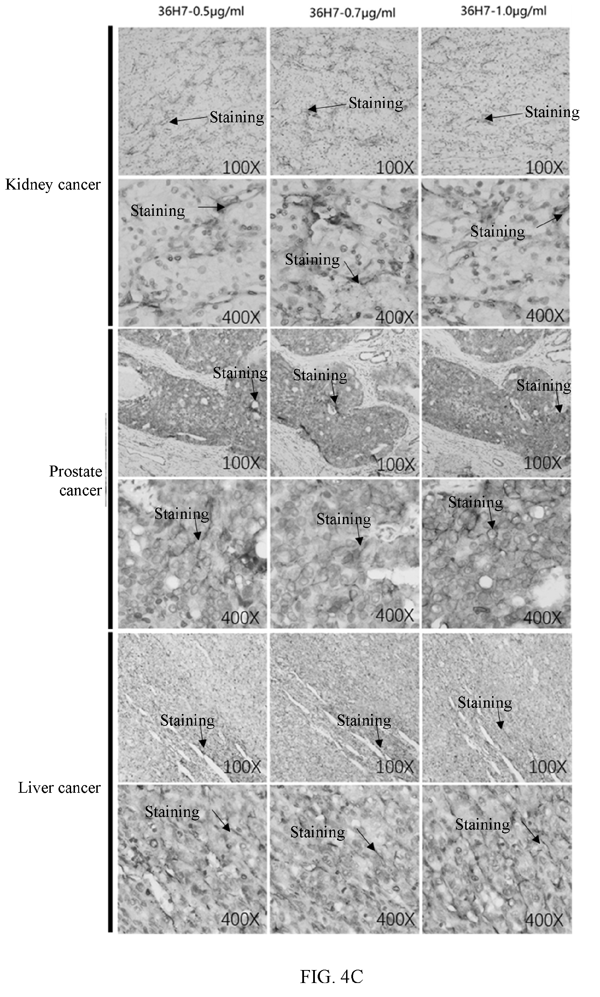

Accordingly, this example further explored the optimal immunohistochemistry experiment concentration of the B7H3 antibody. As shown in FIG. 4C, B7H3-positive kidney cancer, prostate cancer, and liver cancer samples were selected to determine the optimal immunohistochemistry concentration of the B7H3 antibody in this example. Based on the experimental data shown in FIGS. 4A and 4B, immunohistochemistry experiments were performed at concentrations of 1 µg/mL, 0.7 µg/mL, and 0.5 µg/mL. The experimental results showed that at all the concentrations of 1 µg/mL, 0.7 µg/mL, and 0.5 µg/mL, very significant staining in kidney cancer, prostate cancer, and liver cancer samples was produced, with clear cell membrane staining observed. The staining patterns of the B7H3 antibody were highly consistent at the three concentrations. Although the staining intensity of the B7H3 antibody at 1 µg/mL was slightly stronger, it was not significantly distinguishable from that at the other concentrations. To improve detection sensitivity for B7H3 while reducing non-specific background staining, 0.7 µg/mL was selected as the optimal immunohistochemistry experiment concentration of the B7H3 antibody.

### Example 4. B7H3 Antibody Exhibiting High Sensitivity

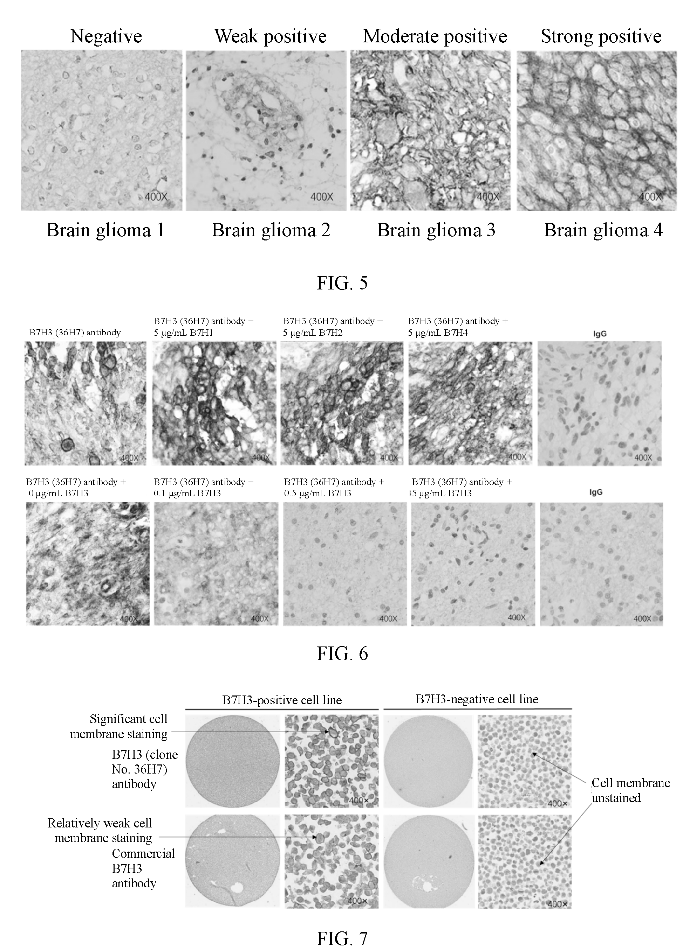

To determine the sensitivity of the B7H3 antibody (clone No. 36H7) in immunohistochemistry experiments, four brain glioma samples with B7H3 expression levels of strong positive, moderate positive, weak positive, and negative, respectively, were selected in this example. Immunohistochemistry experiments were performed on the above four glioma samples with different B7H3 expression levels according to the immunohistochemistry method described in the present disclosure, so as to verify the sensitivity of the B7H3 antibody. As shown in FIG. 5, the staining intensity of the B7H3 antibody (clone No. 36H7) in the four samples was consistent with the expression level trend of B7H3 in the above tissues. Specifically, in the strong positive and moderate positive brain glioma samples, relatively strong staining signals were observed after incubation with the B7H3 antibody, and the staining signal in the strong positive sample was stronger than that in the moderate positive sample. However, in the weak positive sample, the staining signal was significantly reduced after incubation with the B7H3 antibody, and in the negative sample, no staining signal was observed after incubation with the B7H3 antibody. The above data indicate that the B7H3 antibody staining is capable of clearly detecting clinical samples with different B7H3 expression levels, and even for samples with low B7H3 expression, reliable detection and identification can still be achieved.

### Example 5. Specificity of B7H3 (Clone No. 36H7) Antibody for Use in Diagnosis

This example demonstrates that B7H3 can serve as an indicator of disease status. By using the B7H3 antibody of the present application, the B7H3 level in a biological sample can be specifically detected, such that it can be used in the diagnosis of diseases associated with B7H3 expression.

In this example, different concentrations of B7H3 recombinant protein antigen and different types of B7H3 homologous proteins were added to the B7H3 antibody (clone No. 36H7) to simulate the specificity of the B7H3 antibody for the B7H3 protein and the non-specific interference from other homologous proteins, so as to investigate the specificity of the B7H3 antibody in recognizing the B7H3 protein in clinical samples. In this example, a brain glioma clinical sample with high expression of B7H3 was selected for the experiment. As shown in FIG. 6, high concentrations of B7H3 homologous proteins (B7H1, B7H2, and B7H4) were separately added to the B7H3 antibody to form mixed solutions, and immunohistochemistry experiments were performed using the mixed solutions. The results showed that the B7H3 antibody containing B7H3 homologous proteins (B7H1, B7H2, and B7H4) was still capable of detecting B7H3 in the brain glioma sample with high B7H3 expression, presenting a strong staining signal, indicating that the B7H3 homologous proteins (B7H1, B7H2, and B7H4) did not bind to the B7H3 antibody and thus no false positive signals were generated. In addition, different concentrations of B7H3 recombinant protein were separately added to the B7H3 antibody to investigate the effect of the specific binding between the B7H3 antibody and the B7H3 recombinant protein on the detection of B7H3 expression in clinical samples by the B7H3 antibody. As shown in FIG. 6, in the absence of B7H3 recombinant protein addition, the B7H3 antibody was capable of detecting B7H3 in the brain glioma sample, with a relatively strong staining signal. As the amount of B7H3 recombinant protein added increased, the staining signal of the B7H3 antibody gradually decreased and eventually disappeared, becoming consistent with the negative control with no staining signal. This indicated that the B7H3 antibody could bind to the B7H3 protein with high specificity and did not bind to other components in the clinical sample, such that no false positive signals caused by non-specific binding of the B7H3 antibody were observed. The above data indicate that the B7H3 antibody described herein had very high specificity for B7H3 and did not exhibit non-specific binding with other components in clinical samples, thereby avoiding the occurrence of false positive signals. Therefore, the B7H3 antibody described herein exhibits excellent performance in detecting B7H3 expression in clinical samples and can be further developed into a diagnostic reagent for clinical detection of B7H3 expression.

### Example 6. Comparison of Immunohistochemical Performance Between B7H3 Antibody of the Present Application and Commercial Antibody

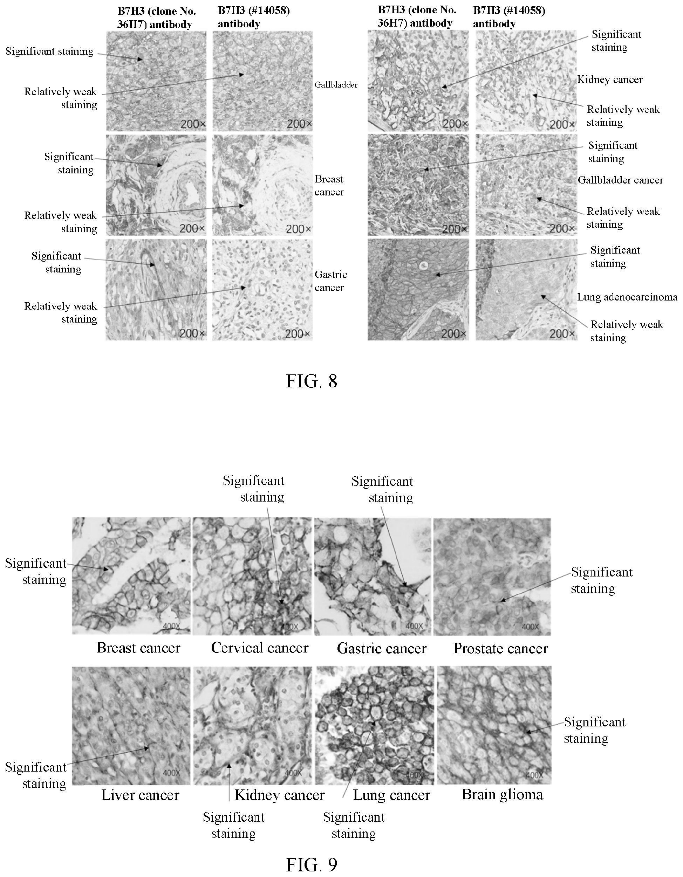

Currently, commercially available monoclonal antibodies targeting B7H3 have been developed. To determine the performance of the B7H3 antibody of the present application, a most widely used B7H3 rabbit monoclonal antibody (Cat. No. CST-B7H3-#14058) was selected in this example and compared with the B7H3 antibody of the present application in terms of immunohistochemical performance, so as to further demonstrate the performance of the B7H3 antibody. In this comparative experiment, the primary antibody concentration and reaction system of the B7H3 rabbit monoclonal antibody (Cat. No. CST-B7H3-#14058) were determined with reference to the instructions and COA of the antibody, and the immunohistochemistry experiment procedures were performed according to the experimental steps described in the present disclosure. The concentration of the B7H3 antibody (clone No. 36H7) was selected as 0.7 µg/mL. In this example, a B7H3-positive cell line, a B7H3-negative cell line, and 6 B7H3-positive clinical samples, including gallbladder, breast cancer, gastric cancer, kidney cancer, gallbladder cancer, and lung adenocarcinoma, were selected for testing. As shown in FIG. 7, both the B7H3 antibody (clone No. 36H7) and the B7H3 (CST-B7H3-#14058) antibody produced significant cellular staining in the B7H3-positive cell line, and no staining was observed in the B7H3-negative cell line, with low background in both cases. However, the staining intensity of the B7H3 antibody (clone No. 36H7) in the B7H3-positive cell line was stronger than that of the B7H3 (CST-B7H3-#14058) antibody, indicating that under their respective optimal reaction conditions, the staining intensity of the B7H3 antibody was higher than that of the B7H3 (CST-B7H3-#14058) antibody. Furthermore, in this example, the B7H3 antibody and the B7H3 (CST-B7H3-#14058) antibody were used to perform immunohistochemistry tests on 6 B7H3-positive clinical samples, including gallbladder, breast cancer, gastric cancer, kidney cancer, gallbladder cancer, and lung adenocarcinoma. As shown in FIG. 8, in all 6 clinical samples, there were significant differences in staining signals between the two antibodies in the same regions of the samples. In kidney cancer, normal gallbladder, and gallbladder cancer samples, the staining intensity of the B7H3 antibody was higher than that of the B7H3 (CST-B7H3-#14058) antibody. In breast cancer, gastric cancer, and pancreatic cancer samples, the staining intensity of the B7H3 antibody was also higher than that of the B7H3 (CST-B7H3-#14058) antibody, and the B7H3 antibody exhibited higher sensitivity. The percentage of immunohistochemically stained B7H3-positive cells detected by the B7H3 antibody was higher than that detected by the B7H3 (CST-B7H3-#14058) antibody. Regions that were not stained by the B7H3 (CST-B7H3-#14058) antibody could be sensitively identified by the B7H3 antibody. The B7H3 antibody demonstrates good sensitivity, thereby facilitating accurate identification of B7H3 expression status, and supporting disease diagnosis and prognosis interpretation.

In summary, compared with the representative commercially available antibody B7H3 (CST-B7H3-#14058), the B7H3 antibody exhibited superior performance. Based on the B7H3 antibody, diagnostic products can be further developed for use in clinical application scenarios such as diagnosis of B7H3-related diseases and prediction of therapeutic efficacy.

### Example 7. Immunoreactivity Detection of B7H3 Antibody

This example demonstrates that the B7H3 antibody of the present application is capable of binding with high affinity to the B7H3 (CD276) protein molecule expressed in tumor tissues and can be used for detecting B7H3 in various tumor tissues.

In this example, representative and high-incidence tumor indications in which B7H3 is highly expressed were selected. These indications include breast cancer, cervical cancer, gastric cancer, prostate cancer, liver cancer, kidney cancer, lung cancer, and brain glioma. Samples from the above tumor indications were used to test the ability of the B7H3 antibody of the present application to detect B7H3 expression in these tumor samples. The immunohistochemistry method used for the B7H3 antibody (clone No. 36H7) in this example was described in detail above.

As shown in FIG. 9, the B7H3 antibody was capable of detecting B7H3 expression in clinical samples of breast cancer, cervical cancer, gastric cancer, prostate cancer, liver cancer, kidney cancer, lung cancer, and brain glioma, with clear cell membrane staining observed. This indicated that despite differences among tumor tissue types, the B7H3 antibody was still capable of recognizing extracellular antigen epitopes of the B7H3 protein in different tumor tissues. These results demonstrate that the B7H3 antibody of the present application has broad applicability in detecting B7H3 expression across various tumor types. The B7H3 antibody of the present application can be used for detecting B7H3 expression in multiple tumor indications, including but not limited to breast cancer, cervical cancer, gastric cancer, prostate cancer, liver cancer, kidney cancer, lung cancer, and brain glioma, thereby providing support for drug efficacy evaluation, diagnosis, treatment, and prognosis of diseases associated with B7H3 expression.

### Example 8. Performance Evaluation of B7H3 Detection Kit (Immunohistochemistry Method)

This example demonstrates that the B7H3 antigen-binding protein described herein can be further prepared into a detection kit, and that the kit can be used as an *in vitro* diagnostic reagent for use in clinical detection of B7H3 expression, thereby supporting drug efficacy evaluation, diagnosis, treatment, and prognosis of diseases associated with B7H3 expression.

The B7H3 detection kit comprising the antigen-binding protein described herein can be used to detect B7H3 expression by an immunohistochemistry method. In addition to the B7H3 antigen-binding protein described herein, the kit may further comprise negative and positive cell line quality control slides and a rabbit isotype antibody IgG. The performance of the detection kit was evaluated on a Leica BOND III automated staining platform (including the BOND III automated stainer, antigen retrieval reagents, and signal enhancement reagents). The specific experimental procedures are shown in Table 3 of this example, and the reagents, consumables, and devices involved in the experiment are listed in Tables 4 and 5 of this example.

**Table 3. Experimental procedures**

| Procedure | Time/min Condition: room temperature 18 °C-27 °C |
|---|---|
| Antigen retrieval | ER2 (EDTA) |
| Hydrogen peroxide blocking | 5 min |
| Primary antibody | 0.7 µg/mL 36H7, incubated at room temperature for 30 min |
| Polymer chromogenic enhancement reagent | Room temperature, 4 min |
| DAB color development | 5 min |
| Hematoxylin | 8 min |

**Table 4. Experimental instruments**

| Name | | Model | | Device No. |
|---|---|---|---|---|
| Immunohistochemistry stainer | | BOND III | | M-047 |

**Table 5. Experimental reagents**

| Reagent name | Cat. No. | Supplier | Host | Property |
|---|---|---|---|---|
| B7H3-36H7 | - | - | Rabbit | Primary antibody |
| Negative control reagent | 3900S | CST | Rabbit | Isotype IgG |
| Bond III signal enhancement reagent | DS9800 | Leica | | HRP-secondary antibody |
| Negative quality control slide | - | - | Human | Jurkat cell line section |
| Positive quality control slide | - | - | Human | U251 cell line section |

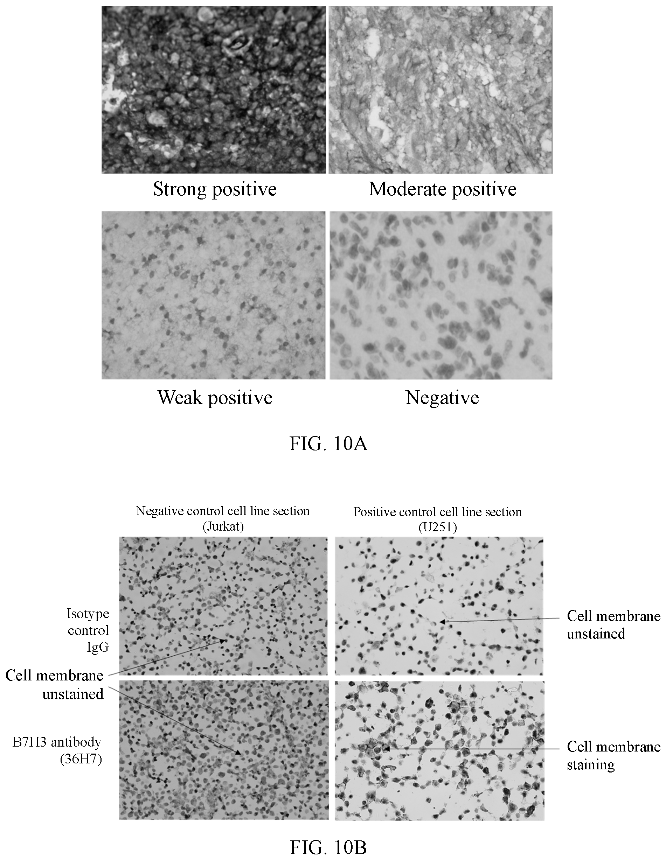

As shown in FIG. 10A, brain glioma samples were used for detection in this example. The kit was capable of accurately detecting B7H3 proteins with different expression levels in brain glioma samples, and the cell membrane staining was relatively clear. The staining results were interpreted based on the percentage of positively stained cells and staining intensity, and the B7H3 expression levels in the tested brain glioma samples were determined as strong positive, moderate positive, weak positive, and negative, respectively, indicating that the kit was capable of detecting B7H3 at different expression levels and exhibited high sensitivity. In addition, as shown in FIG. 10B, in the negative and positive cell line quality control slides, no staining was observed for the negative control IgG antibody. Meanwhile, no staining was observed for the B7H3 antibody in the negative cell line quality control slide, whereas staining was observed in the positive cell line quality control slide, indicating that the selected negative control reagent and the negative and positive quality control slides exhibited good performance and could be used to monitor the entire experimental system, thereby ensuring normal operation of the experiment and obtaining accurate data. Therefore, the kit consisting of the negative and positive cell line quality control slides, the rabbit isotype antibody IgG, and the B7H3 antibody (clone No. 36H7) exhibited good application potential from immunohistochemical detection on the Leica immunohistochemistry staining platform.

## Claims

1. An isolated antigen-binding protein, capable of specifically binding to B7H3, wherein the isolated antigen-binding protein comprises HCDR1, HCDR2, and HCDR3 as defined in any one of the following groups (a) to (d):
a) the HCDR1 comprising the amino acid sequence set forth in SEQ ID NO: 1, the HCDR2 comprising the amino acid sequence set forth in SEQ ID NO: 2, and the HCDR3 comprising the amino acid sequence set forth in SEQ ID NO: 3;
b) the HCDR1 comprising the amino acid sequence set forth in SEQ ID NO: 7, the HCDR2 comprising the amino acid sequence set forth in SEQ ID NO: 8, and the HCDR3 comprising the amino acid sequence set forth in SEQ ID NO: 9;
c) the HCDR1 comprising the amino acid sequence set forth in SEQ ID NO: 12, the HCDR2 comprising the amino acid sequence set forth in SEQ ID NO: 13, and the HCDR3 comprising the amino acid sequence set forth in SEQ ID NO: 3; or
d) the HCDR1 comprising the amino acid sequence set forth in SEQ ID NO: 14, the HCDR2 comprising the amino acid sequence set forth in SEQ ID NO: 15, and the HCDR3 comprising the amino acid sequence set forth in SEQ ID NO: 16.

2. The antigen-binding protein according to claim 1, wherein the antigen-binding protein comprises LCDR1, LCDR2, and LCDR3 as defined in any one of the following groups (a) to (c):
a) the LCDR1 comprising the amino acid sequence set forth in SEQ ID NO: 4, the LCDR2 comprising the amino acid sequence set forth in SEQ ID NO: 5, and the LCDR3 comprising the amino acid sequence set forth in SEQ ID NO: 6;
b) the LCDR1 comprising the amino acid sequence set forth in SEQ ID NO: 10, the LCDR2 comprising the amino acid sequence set forth in SEQ ID NO: 11, and the LCDR3 comprising the amino acid sequence set forth in SEQ ID NO: 6; or
c) the LCDR1 comprising the amino acid sequence set forth in SEQ ID NO: 17, the LCDR2 comprising the amino acid sequence set forth in SEQ ID NO: 18, and the LCDR3 comprising the amino acid sequence set forth in SEQ ID NO: 19.

3. The isolated antigen-binding protein according to any one of claims 1 to 2, wherein the isolated antigen-binding protein comprises a heavy chain variable region (VH), and the VH comprises the amino acid sequence set forth in SEQ ID NO: 20.

4. The isolated antigen-binding protein according to any one of claims 1-3, wherein the isolated antigen-binding protein comprises a light chain variable region (VL), and the VL comprises the amino acid sequence set forth in SEQ ID NO: 21.

5. The isolated antigen-binding protein according to any one of claims 1-4, wherein the isolated antigen-binding protein comprises a heavy chain variable region having the amino acid sequence set forth in SEQ ID NO: 20 and a light chain variable region having the amino acid sequence set forth in SEQ ID NO: 21.

6. The isolated antigen-binding protein according to any one of claims 1-5, wherein the isolated antigen-binding protein comprises a heavy chain constant region, and the heavy chain constant region may be derived from a human or rabbit antibody heavy chain constant region.

7. The isolated antigen-binding protein according to any one of claims 1-6, wherein the isolated antigen-binding protein comprises a heavy chain constant region, and the heavy chain constant region may be derived from an IgG heavy chain constant region.

8. The isolated antigen-binding protein according to any one of claims 1-7, wherein the heavy chain constant region thereof may comprise the amino acid sequence set forth in SEQ ID NO: 22.

9. The isolated antigen-binding protein according to any one of claims 1-8, wherein the isolated antigen-binding protein comprises a light chain constant region, and the light chain constant region thereof may be derived from a human or rabbit antibody light chain constant region.

10. The isolated antigen-binding protein according to any one of claims 1-9, wherein the isolated antigen-binding protein comprises a light chain constant region, and the light chain constant region thereof may be derived from a human or rabbit κ chain or λ chain.

11. The isolated antigen-binding protein according to any one of claims 1-10, wherein the light chain constant region thereof may comprise the amino acid sequence set forth in SEQ ID NO: 23.

12. The isolated antigen-binding protein according to any one of claims 1-11, wherein the isolated antigen-binding protein comprises an antibody heavy chain HC, and the HC comprises a heavy chain having the amino acid sequence set forth in SEQ ID NO: 24.

13. The isolated antigen-binding protein according to any one of claims 1-12, wherein the isolated antigen-binding protein comprises an antibody light chain LC, and the LC comprises a light chain having the amino acid sequence set forth in SEQ ID NO: 25.

14. The isolated antigen-binding protein according to any one of claims 1-13, wherein the isolated antigen-binding protein comprises an antibody or an antigen-binding fragment thereof.

15. The isolated antigen-binding protein according to claim 14, wherein the antigen-binding fragment comprises a Fab, a Fab', an Fv fragment, an F(ab')₂, an F(ab)₂, an scFv, a di-scFv, and/or a dAb.

16. The isolated antigen-binding protein according to claim 14, wherein when the antigen-binding protein is an scFv, the heavy and light chain variable regions thereof may comprise a linker peptide therebetween.

17. The isolated antigen-binding protein according to any one of claims 1-16, wherein the antibody is one or more selected from the group consisting of: a monoclonal antibody, a rabbit antibody, a chimeric antibody, a humanized antibody, a recombinant antibody, a single-chain antibody, a bifunctional antibody, a trifunctional antibody, and a tetrafunctional antibody.

18. The isolated antigen-binding protein according to claim 17, wherein the chimeric antibody comprises a human, goat, monkey, dog, mouse, or rabbit chimeric antibody.

19. The isolated antigen-binding protein according to any one of claims 1-18, wherein the antigen-binding protein is an IgG antibody.

20. The isolated antigen-binding protein according to any one of claims 1-19, wherein the antigen-binding protein is labeled.

21. The isolated antigen-binding protein according to claim 20, wherein the label comprises a fluorescent label, an enzymatic label, a biotin label, or a radioactive label.

22. A polypeptide molecule, comprising the isolated antigen-binding protein according to any one of claims 1-21.

23. A chimeric antigen receptor, comprising the isolated antigen-binding protein according to any one of claims 1-21.

24. An immunoconjugate, comprising the isolated antigen-binding protein according to any one of claims 1-21.

25. A nucleic acid molecule, encoding the isolated antigen-binding protein according to any one of claims 1-21, the polypeptide molecule according to claim 22, or the chimeric antigen receptor according to claim 23.

26. A vector, comprising the nucleic acid molecule according to claim 25.

27. A cell, which expresses the antigen-binding protein according to any one of claims 1-21, the polypeptide molecule according to claim 22, or the chimeric antigen receptor according to claim 23, or which comprises the nucleic acid molecule according to claim 25, or the vector according to claim 26.

28. A pharmaceutical composition, comprising the isolated antigen-binding protein according to any one of claims 1-21, the polypeptide molecule according to claim 22, the chimeric antigen receptor according to claim 23, the immunoconjugate according to claim 24, the nucleic acid molecule according to claim 25, the vector according to claim 26, and/or the cell according to claim 27, and a pharmaceutically acceptable carrier.

29. A reagent, comprising the isolated antigen-binding protein according to any one of claims 1-21, the polypeptide molecule according to claim 22, the chimeric antigen receptor according to claim 23, the immunoconjugate according to claim 24, the nucleic acid molecule according to claim 25, the vector according to claim 26, the cell according to claim 27, and/or the pharmaceutical composition according to claim 28.

30. An assay plate, comprising the isolated antigen-binding protein according to any one of claims 1-21, the polypeptide molecule according to claim 22, the chimeric antigen receptor according to claim 23, the immunoconjugate according to claim 24, the nucleic acid molecule according to claim 25, the vector according to claim 26, the cell according to claim 27, and/or the pharmaceutical composition according to claim 28.

31. A kit, comprising the isolated antigen-binding protein according to any one of claims 1-21, the polypeptide molecule according to claim 22, the chimeric antigen receptor according to claim 23, the immunoconjugate according to claim 24, the nucleic acid molecule according to claim 25, the vector according to claim 26, the cell according to claim 27, the pharmaceutical composition according to claim 28, the reagent according to claim 29, and/or the assay plate according to claim 30.

32. The kit according to claim 31, wherein the kit comprises a negative control reagent, a negative control material, a quality control reagent, and/or a quality control material.

33. A method for preparing the isolated antigen-binding protein according to any one of claims 1-21.

34. The method according to claim 33, wherein the method comprises the following steps:
a) culturing the cell according to claim 27; and/or
b) isolating an antigen-binding protein from a culture, wherein the antigen-binding protein is the antigen-binding protein according to any one of claims 1-21.

35. A method for detecting B7H3 in a sample, comprising using the isolated antigen-binding protein according to any one of claims 1-21, the polypeptide molecule according to claim 22, the chimeric antigen receptor according to claim 23, the immunoconjugate according to claim 24, the nucleic acid molecule according to claim 25, the vector according to claim 26, the cell according to claim 27, the pharmaceutical composition according to claim 28, the reagent according to claim 29, the assay plate according to claim 30, and/or the kit according to claim 32.

36. Use of the isolated antigen-binding protein according to any one of claims 1-21, the polypeptide molecule according to claim 22, the chimeric antigen receptor according to claim 23, the immunoconjugate according to claim 24, the nucleic acid molecule according to claim 25, the vector according to claim 26, the cell according to claim 27, and/or the pharmaceutical composition according to claim 28 in preparing a medicament for use in preventing and/or treating a disease and/or condition.

37. The use according to claim 36, wherein the disease and/or condition includes a B7H3-related disease and/or condition.

38. The use according to claim 37, wherein the disease and/or condition includes a tumor.

39. The use according to any one of claims 36 and 37, wherein the disease and/or condition includes adrenocortical carcinoma, bladder cancer, breast cancer, cholangiocarcinoma, colorectal cancer, lymphoma, esophageal cancer, brain glioma, neuroblastoma, head and neck squamous cell carcinoma, kidney cancer, liver cancer, lung cancer, ovarian cancer, pancreatic cancer, prostate cancer, sarcoma, melanoma, gastric cancer, thymus cancer, endometrial cancer, and/or cervical cancer.

40. Use of the isolated antigen-binding protein according to any one of claims 1-21, the polypeptide molecule according to claim 22, the chimeric antigen receptor according to claim 23, the immunoconjugate according to claim 24, the nucleic acid molecule according to claim 25, the vector according to claim 26, the cell according to claim 27, and/or the pharmaceutical composition according to claim 28 in preparing a detection reagent.

41. The use according to claim 40, wherein the detection reagent is used for:
a) detecting B7H3 in a sample;
b) detecting endogenous B7H3 protein in tumor cells;
c) detecting tumor cells expressing B7H3;
d) detecting the expression level of B7H3 in a biological sample from a patient to predict or determine prognosis and/or progression of a disease and/or condition;
e) detecting the expression level of B7H3 in a biological sample from a patient to determine a patient population for administration of a B7H3-targeted therapy, wherein when the expression level of B7H3 is medium or high, the B7H3-targeted therapy is administered to the patient;
f) detecting the expression level of B7H3 in a biological sample from a patient to predict or determine efficacy of a B7H3-targeted therapy administered to the patient, wherein when the expression level of B7H3 is medium or high, it indicates that the B7H3-targeted therapy is therapeutically effective; and/or
g) detecting the expression level of B7H3 in a biological sample from a patient to modify a treatment regimen for the patient, wherein when the expression level of B7H3 is medium or high, the treatment regimen is modified to comprise a B7H3-targeted therapy.

42. The use according to any one of claims 40 and 41, wherein the detection comprises immunohistochemical detection, immunofluorescence detection, flow cytometry detection, enzyme-linked immunosorbent detection, protein/peptide microarray detection, immunoblot detection, bead-based immunoassay, and/or microfluidic immunoassay.

43. The use according to any one of claims 40-42, wherein the patient is a patient with a tumor or cancer.

44. The use according to any one of claims 40-43, wherein the patient is a patient with a B7H3-related tumor or cancer.

45. The use according to any one of claims 40-44, wherein the cancer or tumor includes: bladder cancer, breast cancer, cholangiocarcinoma, colorectal cancer, lymphoma, esophageal cancer, brain glioma, neuroblastoma, head and neck squamous cell carcinoma, kidney cancer, liver cancer, lung cancer, ovarian cancer, pancreatic cancer, prostate cancer, sarcoma, melanoma, gastric cancer, thymus cancer, endometrial cancer, and/or cervical cancer.
